(19) **Europäisches Patentamt**
**European Patent Office**
**Office européen des brevets**

(11) **EP 4 556 905 A1**

(12) **EUROPEAN PATENT APPLICATION**

(43) Date of publication:
**21.05.2025 Bulletin 2025/21**

(21) Application number: **23383165.0**

(22) Date of filing: **15.11.2023**

(51) International Patent Classification (IPC):
***G01N 33/00*** *(2006.01)* ***G01N 33/68*** *(2006.01)*

(52) Cooperative Patent Classification (CPC):
**G01N 33/6872;** G01N 24/08; G01N 2333/4703;
G01N 2500/02; G01R 33/465

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Agencia Estatal Consejo Superior
De Investigaciones Científicas
28006 Madrid (ES)**

(72) Inventors:
• **Antón, Rosa**
**Madrid (ES)**

• **Pantoja-Uceda, David**
**Madrid (ES)**
• **Treviño, Miguel Ángel**
**Madrid (ES)**
• **Oroz Garde, Francisco Javier**
**Madrid (ES)**

(74) Representative: **Pons
Glorieta Rubén Darío 4
28010 Madrid (ES)**

Remarks:
The complete document including Reference
Table(s) and the Sequence Listing(s) can be
downloaded from the EPO website

(54) **METHOD FOR THE SCREENING OF ACTIVE AGENTS THAT INHIBIT CHANGES IN THE STRUCTURE OF A TRANSCRIPTION FACTOR COMPRISING AMINO ACID REPEAT MUTATIONS**

(57) The present invention refers to an in vitro method for the screening of active agents that inhibit changes in secondary structure in a transcription factor, wherein the changes in secondary structure are responsible for the formation of aggregates of said transcription factor, and wherein the method is based on NMR. The invention also refers the use of a transcription factor that comprises an amino acid repeat mutation, or a fragment thereof, for the in vitro screening of an active agent that inhibits changes in secondary structure in the transcription factor by NMR.

**EP 4 556 905 A1**

## Description

The invention relates to methods for the *in vitro* screening of active agents that modulate protein structure changes responsible for the formation of protein aggregates, therefore the present invention is within the biotechnology field.

## BACKGROUND ART

Abnormal trinucleotide repeat expansions alter protein conformation causing malfunction and contribute to a significant number of incurable human diseases. Eukaryotic proteomes contain abundant stretches of sequentially repeated aminoacids or homorepeats, being polyglutamine, polyasparagine and polyalanine the most prevalent. Although the function of homorepeats is still unclear, they are presumed to play roles in protein structure and interactomes, acting as polymorphic adaptors in a length- and boundaries-dependent manner. Recombination between two mispaired alleles can cause expansions as well as contractions in homorepeats (Matera, I. et al. J Med Genet 41, 373-80 (2004)). Indeed, the aberrant increments of consecutive glutamine residues by trinucleotide repeat expansion (TRE) mutations were linked to multiple human diseases more than 30 years ago (Chavali, S., Singh, A.K., Santhanam, B. & Babu, M.M. Nature Reviews Chemistry 4, 420-434 (2020)).

It is essential to understand the molecular mechanisms of TRE-based diseases to develop efficient therapeutics. Considering that the structural conversions induced by the TREs have a profound impact in protein malfunction, intense research was dedicated to describe the structure of TRE mutants. However, due to their challenging nature, structural descriptions of homorepeat-containing proteins are currently scarce. Scarce structural insights available on disease-related homorepeat expansions hinder the design of effective therapeutics, and TRE-based diseases remain currently uncurable.

## DESCRIPTION OF THE INVENTION

The present invention discloses an *in vitro* method that allows to determine the capacity of active agents for modulating the appearance of protein structures in TRE mutants that are directly responsible for the formation of protein aggregates that lead to protein loss of function. Therefore, the method described in the present invention allows to screen active agents that may be useful in the treatment of TRE-based diseases associated with the formation of protein aggregates.

In particular, the method presented herein comprises a platform of Nuclear Magnetic Resonance (NMR) spectroscopy experiments that enable to detect incipient disordered protein structures which are triggered by TRE mutations. These low-populated, alternative structures are highly dynamic and can only be detected by solution NMR spectroscopy. This NMR methodology, in combination with light scattering procedures demonstrate that the alternative structures are responsible for protein aggregation in TRE mutants, which is a major molecular mechanism that explains protein pathophysiology in TRE-based diseases. The present inventors also demonstrate here that tackling the incipient dynamic structures triggered by the TRE mutations blocks protein aggregation. Taking advantage of the precise structural information provided by NMR spectroscopy, it is possible to target specific protein regions to be used as templates for the design of compounds that modulate aggregation, providing a significant advantage towards other screening methods.

Thus, a first aspect of the present invention is related to an *in vitro* method for the screening of an active agent that inhibits changes in secondary structure in a transcription factor, wherein the changes in secondary structure are responsible for the formation of aggregates of said transcription factor, hereinafter "the method of the invention", wherein said method comprises the following steps:

a) determining by NMR spectroscopy any change in secondary structure in a transcription factor, or a fragment thereof,

- wherein said transcription factor comprises an amino acid repeat mutation,
- wherein the amino acid repeat mutation gives rise to changes at the secondary structure level, and
- wherein the changes in secondary structure are responsible for the formation of aggregates,

b) determining by NMR spectroscopy any change in secondary structure in a transcription factor used in step a), or a fragment thereof, upon incubation with an active agent, and

wherein the active agent is selected as an inhibitor of the changes in secondary structure that are responsible for the formation of aggregates, if there is a reduction in the changes in secondary structure determined in step b) compared to the changes in secondary structure determined in step a).

In the present invention, the term "transcription factor" refers to a protein that is involved in the regulation of gene expression in cells. Transcription factors may have a positive effect on gene expression and are therefore sometimes referred to as "activators" or "transcription activators". In another embodiment, the transcription factor may perform negative gene expression and is therefore sometimes referred to as a "repressor" or "transcription repressor". The correct function of transcription factors is essential for exerting an appropriate control over the expression of genes, leading to an adequate cell function. In mammals, some genes coding for transcription factors may comprise mutations that give rise to the defective function of said transcription factor. Subsequently, the incorrect function of mutant transcription factors may result in the development of a disease in the subject. Therefore, it is needed to study and understand the molecular mechanisms related to the malfunction of mutant transcription factors in order to develop new therapeutic strategies to treat diseases related.

There are transcription factors that regulate the expression of different genes leading to the differentiation and function of specific cell linages being part of a complex organism, such as a human being, through its development, wherein said transcription factors are referred to as developmental transcription factors. In mammals, some genes coding for developmental transcription factors comprise trinucleotide repeats that may be located within coding regions or non-coding regions, such as introns. Trinucleotide repeats comprised in coding regions give rise to amino acid repeats in the resulting transcription factor when the gene is expressed. As it is known for the skilled person, there are several diseases or disorders such as developmental or neurological disorders that include, but are not limited to, congenital central hypoventilation syndrome, Huntington's disease, spinocerebellar ataxia type 1 or dentatorubral-pallidoluysian atrophy, wherein genes coding developmental transcriptional factors comprise trinucleotide repeat expansions mutations.

Thus, in a preferred embodiment of the method of the invention the transcription factor is a developmental transcription factor. In the present invention, the term "developmental transcription factor" refers to a transcription factor that is involved in the gene regulation, specifically during the development of a subject. Examples of developmental transcription factors include, without limitation, ARX, FOXL2, RUNX2, PHOX2B, HOXA13, ZIC2, HOXD13, ARX or SOX3.

In the present invention the term "subject" refers to a mammal, more preferably wherein the mammal is selected from a human being and a non-human mammal. More preferably, the term subject refers to a human being, thus in some preferred embodiments the transcription factor is a human transcription factor.

Trinucleotide repeat expansions (TRE) refer to a type of genetic mutation wherein the nucleotide sequence of a gene comprises excessive trinucleotide repeats (e.g., trinucleotide repeats such as CAG) that exceed the normal stable threshold for the gene comprising said mutation in a subject. TREs may be located in coding regions of a gene (i.e. exons) or located in non-coding regions (i.e. introns) of the mutated gene. In the case of trinucleotide repeats located in coding exons of a determined gene, the resulting protein comprises an aminoacid repeat mutation resulting from the transcription and translation of the expanded codons. Therefore, when the mutated gene is expressed, the resulting protein (e.g. a developmental transcription factor) comprises an aminoacid repeat mutation made of a segment of identical aminoacids that is an expansion of an homomeric amino acid repeat determined by the number of TREs in the coding exon. Several mechanisms have been proposed to explain the pathogenicity of TREs, such as protein deregulation or loss-of-function, cellular mislocalization, dominant-negative effects or a toxic gain of function (Pirone, L. et al. FEBS J 286, 2505-2521 (2019)).

As it has been determined by the present inventors, the phase transition, specifically, liquid-solid transition, of a transcription factor was affected when the number of amino acid residues of an amino acid repeat region was increased over a determined threshold. The mentioned threshold refers to the number of residues that is found in the amino acid repeat of a wild-type or non-pathogenic form of a protein, and thus the protein having a number of aminoacid repeat residues equal or lower than the threshold, keeps its structural and functional features. Specifically, the inventors observed that the increase (or expansion) of the residues in the amino acid repeat promoted a rapid liquid-solid transition of a transcription factor when compared to the wild-type or non-pathogenic transcription factor. Moreover, the inventors found that the amino acid repeat mutation promoted the formation of protein aggregates compared to the wild-type or non-pathogenic transcription factor.

The term "phase transitions" refers to a density transition in multicomponent fluids or condensation process by which an entity, made up of biomolecules such as proteins, that is not bound by a membrane that concentrates specific types of biomolecules is formed. Phase transitions can occur by percolation or networking transitions enabled by multivalent interactions, which can be coupled to phase separation. Therefore, phase separation coupled to percolation leads to viscoelastic condensates. In this scenario, the density of the transcription factor that comprises an aminoacid repeat mutation in the dense phase or viscoelastic condensate will be high, while it will be low in the dilute phase, meaning that the transcription factor that comprises an amino acid repeat mutation undergoes a liquid to solid transition that favours the formation of aggregates/condensates (Anja Mittag, Rohit V. Pappu, A conceptual framework for understanding phase separation and addressing open questions and challenges. Molecular Cell, Volume 82, Issue 12, 2022, Pages 2201-221).

Phase transitions and/or formation of aggregates may be promoted by changes in secondary structure of a protein. The secondary structure of a protein, i.e. a transcription factor, refers to the local spatial conformation of the polypeptide backbone, that arises and is stabilized by hydrogen bonds between atoms of the amino acid residues. The main secondary structures of a protein include α-helix, β-sheets and turns. The secondary structure of a protein may change due to the mutations in the amino acid sequence, such as amino acid repeats mutations, leading to conformational changes between α-helix, β-sheets, turns and disordered structures (polypeptide segments that do not contain sufficient hydrophobic amino acids to mediate co-operative folding). Thus, in the present invention the term "change in secondary structure in a transcription factor" refers to a change or transition from a type of secondary structure to another type of secondary structure conformation in a region of the amino acid sequence of a transcription factor. Therefore, changes in secondary structure represent incipient structures. Changes in secondary structure may comprise the change from α-helix to β-sheet, from α-helix to a turn, from α-helix to a disordered structure, from β-sheet to α-helix, from β-sheet to a turn, from β-sheet to a disordered structure, from disordered structure to α-helix, from disordered structure to β-sheet, from disordered structure to a turn, from a turn to α-helix, from a turn to β-sheet, from a turn to disordered structure, or any combination thereof.

The term "change responsible for the formation of aggregates" refers to a change at secondary structure level that promotes the formation of aggregates in a protein, i.e. a transcription factor. As used in the present invention, the terms "aggregate", "condensate", "protein aggregate", "protein condensate" or "pathogenic aggregate", used interchangeably, refer to toxic, misfolded and aggregated variants of proteins or polypeptides present in the body of a subject, whose function is altered. Protein aggregates may be formed intra or extracellularly by non-covalent interactions of the amino acids of different protein molecules that conform the aggregate.

In another preferred embodiment, the method of the invention further comprises a step c) that comprises determining the formation of aggregates of the transcription factor, or a fragment thereof, of the step a) and step b). The determinations of the formation of aggregates of the transcription factor, or a fragment thereof, of the step a) and step b), are performed separately.

Step c) in the method of the invention allows to confirm the correlation between any change in secondary structure in a transcription factor, or a fragment thereof, and the formation of aggregates. Thus, the data retrieved from NMR measurements in combination with data form step c), allow to establish which part or parts of the transcription factor interact with the active agent, being this information relevant for the development and a more accurate selection of new active agents that inhibit formation of aggregates of transcription factors comprising aminoacid repeat mutations.

In a more preferred embodiment of the method of the invention, the step c) is carried out by light scattering, optical microscopy, electron microscopy, atomic force microscopy, analytical ultracentrifugation, chromatography and/or mass spectrometry, or any combination thereof.

In the present invention the wild-type or non-pathogenic transcription factor refers to a transcription factor comprising an amino acid repeat with a number of residues that does not exceed a threshold, and wherein the wild-type or non-pathogenic transcription factor does not show an altered liquid-solid transition and does not form aggregates. As the skilled person knows, the threshold that defines when a transcription factor comprises an aminoacid repeat mutation depends on the transcription factor and the disease related to it. Table 1 includes, as non-limiting examples, a list of diseases related to genes that comprise TREs mutations. The table 1 also includes the mutation of each gene in terms of the number of trinucleotide repeats considered as a pathogenic mutation.

Table 1. Developmental transcription factors with sequential amino acid expansions and related diseases. Mutation refers to those mutations related with human disease. DRPLA: Dentatorubral pallidoluysian atrophy; HD: Huntington's disease; HDL2: Huntington disease-like 2; SBMA: Spinal and bulbar muscular atrophy; SCA1: Spinocerebellar ataxia type 1; SCA2: Spinocerebellar ataxia type 2; SCA3: Spinocerebellar ataxia type 3; SCA6: Spinocerebellar ataxia type 6; SCA7: Spinocerebellar ataxia type 7; SCA8: Spinocerebellar ataxia type 8; SCA17: Spinocerebellar ataxia type 17; OPMD: Oculopharyngeal muscular dystrophy; XLMR: X-linked mental retardation; BPES: Blepharophimosis, ptosis, and epicanthus inversus syndrome; CCD: Cleidocranial Dysplasia; CCHS: Congenital central hypoventilation syndrome; HFGS: Hand-foot-genital syndrome; HPE: Holoprosencephaly; SPD: Sensory processing disorder; XLAG: X-linked lissencephaly with abnormal genitalia; XLMRGHD: X-linked mental retardation with growth hormone deficit.

| Disease | Gene | expansion | Mutation | genbank | Uniprot_ID |
|---|---|---|---|---|---|
| DRPLA | ATN1 | Polyglutamine | $(CAG)_{>48}$ | D31840.1 | P54259 |
| HD | HTT | Polyglutamine | $(CAG)_{>36}$ | L12392.1 | P42858 |
| HDL2 | JPH3-AS | Polyglutamine | $(CAG)_{>41}$ | AB042636.1 | Q8WXH2 |
| SBMA | AR | Polyglutamine | $(CAG)_{>38}$ | M20132.1 | P10275 |
| SCA1 | ATXN1 | Polyglutamine | $(CAG)_{>40}$ | X79204.1 | P54253 |
| SCA2 | ATXN2 | Polyglutamine | $(CAG)_{>33}$ | U70323.1 | Q99700 |
| SCA3 | ATXN3 | Polyglutamine | $(CAG)_{>52}$ | S75313.1 | P54252 |

(continued)

| Disease | Gene | expansion | Mutation | genbank | Uniprot_ID |
|---------|------|-----------|----------|---------|------------|
| SCA6 | CACNA1A | Polyglutamine | $(CAG)_{>20}$ | AF004883.1 | O00555 |
| SCA7 | ATXN7 | Polyglutamine | $(CAG)_{>36}$ | 4J000517.1 | O15265 |
| SCA8 | ATXN8 | Polyglutamine | $(CAG)_{80-250}$ | DQ641254.1 | Q156A1 |
| SCA17 | TBP | Polyglutamine | $(CAG)_{>43}$ | X54993.1 | P20226 |
| OPMD | PABPN1 | Polyalanine | $(GCN)_{11-17}$ | AF026029.1 | Q86U42 |
| XLMR | ARX | Polyalanine | $(GCN)_{20\ or\ 23}$ | 4Y038071.1 | Q96QS3 |
| BPES | FOXL2 | Polyalanine | $(GCN)_{20\ or\ 23}$ | AF301906.1 | P58012 |
| CCD | RUNX2 | Polyalanine | $(GCN)_{27}$ | AH005498.2 | Q13950 |
| CCHS | PHOX2B | Polyalanine | $(GCN)_{25-33}$ | D82344.1 | Q99453 |
| HFGS | HOXA13 | Polyalanine | $(GCN)_{22-29}$ | U82827.1 | P31271 |
| HPE | ZIC2 | Polyalanine | $(GCN)_{25}$ | AF104902.1 | O95409 |
| SPD | HOXD13 | Polyalanine | $(GCN)_{22-29}$ | AH006690.2 | P35453 |
| XLAG | ARX | Polyalanine | $(GCN)_{20}$ | AY038071.1 | Q96QS3 |
| XLMRGHD | SOX3 | Polyalanine | $(GCN)_{20}$ | X71135.1 | P41225 |

The threshold from which an expansion of amino acids in a transcription factor leads to a rapid liquid-solid transition and/or the formation of aggregates, may be determined by any known method for the skilled person, such as light scattering, optical microscopy, electron microscopy, atomic force microscopy, analytical ultracentrifugation, chromatography and mass spectrometry. The aforementioned threshold may be determined as in the experiments of the present application.

In the present invention, the term "amino acid repeat mutation" refers to an amino acid repeat in a protein or peptide that comprises a sequential expansion of the amino acid residues compared to the wild-type or non-pathogenic protein or peptide. The aminoacid repeat mutation is the consequence of a TRE mutation in the coding exons of the gene. In the case of transcription factors that comprise an amino acid repeat mutation, said mutation promotes the formation of protein aggregates leading to the malfunction of the transcription factor and therefore it cannot exert its gene regulation function. In the present invention the amino acid repeat is an homorepeat, meaning that the amino acid residues of the repeat are identical amino acid residues. The expansion of the amino acid repeat may comprise an addition of +2, +3, +4, +5, +6, or more identical amino acid residues, to the amino acid repeat threshold found in the wild-type or non-pathogenic protein or peptide, preferably being a developmental transcription factor. In the present invention the terms "amino acid repeat mutation" and "aminoacid expansion" are equivalent and may be used interchangeably.

The most frequent TREs are those coding for hydrophobic aliphatic or hydrophilic polar uncharged aminoacids. Examples of TREs coding for hydrophobic aliphatic or hydrophilic polar uncharged aminoacids, include GCN and CAG, respectively.

According to the human genetic code CAG codes for glutamine, while GCN codes for alanine.

Thus, in a preferred embodiment of the method of the invention, the transcription factor comprises an amino acid repat mutation of a hydrophobic aliphatic amino acid or a hydrophilic polar uncharged amino acid; more preferably, the hydrophobic aliphatic amino acid is alanine and wherein the uncharged amino acid is glutamine.

According to the results obtained by the present inventors, amino acid expansions promote changes in the secondary structure of a transcription factor that leads to the formation of aggregates, and wherein the formation of aggregates hinders the transcription function of transcription factors.

In a preferred embodiment of the method of the invention, the amino acid repeat of the transcription factor comprises at least 10 consecutive identical residues. In a more preferred embodiment of the method of the invention, the amino acid repeat of the transcription factor comprises from 10 to 250 consecutive identical residues.

In another preferred embodiment of the method of the invention, the transcription factor is a developmental transcription factor selected from the list consisting of ATN1, HTT, JPH3-AS, AR, ATXN1, ATXN2, ATXN3, CACNA1A, ATXN7, ATXN8, TBP, PABPN1, ARX, FOXL2, RUNX2, PHOX2B, HOXA13, ZIC2, HOXD13, ARX and SOX3.

In the context of the present invention, fragments of transcription factor that comprises an amino acid repeat mutation are also encompassed, provided that such fragments show rapid liquid-solid transition, and therefore, they show a higher tendency to form aggregates in comparison to the same fragment from the wild-type transcription factor. As used in the present invention, the term "fragment" refers to a protein or polypeptide with one or more amino acids (or nucleotides) missing from the ends of its sequence, such as the amino and/or carboxyl terminus of the protein or polypeptide wherein; preferably, the fragment is a fragment of a transcription factor with an amino acid repeat mutation and said fragment shows a liquid-solid transition and formation of aggregates as the complete transcription factor. The liquid-solid transition and the formation of aggregates of a transcription factor, or a fragment thereof, with an amino acid repeat mutation may be determined as in the examples of the present application. In the present invention, the fragment comprises the amino acid residues corresponding to the amino acid repeat mutation.

In another preferred embodiment of the method of the invention, the developmental transcription factor that comprises an aminoacid repeat mutation is ATN1, preferably wherein the amino acid repeat comprises more than 48 glutamine residues. Thus, the nucleotide sequence of the mutant gene that codes for the mutant ATN1 protein, comprises a trinucleotide repeat having more than 48 consecutive CAG codons in a coding exon. The wild-type or non-pathogenic form of ATN1 comprises an amino acid repeat having no more than 48 glutamine residues, and consequently the gene coding for the wild-type or non-pathogenic form of ATN1 comprises a trinucleotide repeat having no more than 48 consecutive CAG codons.

In another preferred embodiment of the method of the invention, the developmental transcription factor that comprises an aminoacid repeat mutation is HTT, preferably wherein the amino acid repeat comprises more than 36 glutamine residues. Thus, the nucleotide sequence of the mutant gene that codes for the mutant HTT protein, comprises a trinucleotide repeat having more than 36 consecutive CAG codons in a coding exon. The wild-type or non-pathogenic form of HTT comprises an amino acid repeat having no more than 36 glutamine residues, and consequently the nucleotide sequence of the gene coding for the wild-type or non-pathogenic form of HTT comprises a trinucleotide repeat having no more than 36 consecutive CAG codons.

In another preferred embodiment of the method of the invention, the developmental transcription factor that comprises an aminoacid repeat mutation is JPH3-AS, preferably wherein the amino acid repeat comprises more than 41 glutamine residues. Thus, the nucleotide sequence of the mutant gene that codes for the mutant JPH3-AS protein, comprises a trinucleotide repeat having more than 41 consecutive CAG codons in a coding exon. The wild-type or non-pathogenic form of JPH3-AS comprises an amino acid repeat having no more than 41 glutamine residues, and consequently the nucleotide sequence of the gene coding for the wild-type or non-pathogenic form of JPH3-AS comprises a trinucleotide repeat having no more than 41 consecutive CAG codons.

In another preferred embodiment of the method of the invention, the developmental transcription factor that comprises an aminoacid repeat mutation is AR, preferably wherein the amino acid repeat comprises more than 38 glutamine residues. Thus, the nucleotide sequence of the mutant gene that codes for the mutant AR protein, comprises a trinucleotide repeat having more than 38 consecutive CAG codons in a coding exon. The wild-type or non-pathogenic form of AR comprises an amino acid repeat having no more than 38 glutamine residues, and consequently the nucleotide sequence of the gene coding for the wild-type or non-pathogenic form of AR comprises a trinucleotide repeat having no more than 38 consecutive CAG codons.

In another preferred embodiment of the method of the invention, the developmental transcription factor that comprises an aminoacid repeat mutation is ATXN1, preferably wherein the amino acid repeat comprises more than 40 glutamine residues. Thus, the nucleotide sequence of the mutant gene that codes for the mutant ATXN1 protein, comprises a trinucleotide repeat having more than 40 consecutive CAG codons in a coding exon. The wild-type or non-pathogenic form of ATXN1 comprises an amino acid repeat having no more than 40 glutamine residues, and consequently the nucleotide sequence of the gene coding for the wild-type or non-pathogenic form of ATXN1 comprises a trinucleotide repeat having no more than 40 consecutive CAG codons.

In another preferred embodiment of the method of the invention, the developmental transcription factor that comprises an aminoacid repeat mutation is ATXN2, preferably wherein the amino acid repeat comprises more than 33 glutamine residues. Thus, the nucleotide sequence of the mutant gene that codes for the mutant ATXN2 protein, comprises a trinucleotide repeat having more than 33 consecutive CAG codons in a coding exon. The wild-type or non-pathogenic form of ATXN2 comprises an amino acid repeat having no more than 33 glutamine residues, and consequently the nucleotide sequence of the gene coding for the wild-type or non-pathogenic form of ATXN2 comprises a trinucleotide repeat having no more than 33 consecutive CAG codons.

In another preferred embodiment of the method of the invention, the developmental transcription factor that comprises an aminoacid repeat mutation is ATXN3, preferably wherein the amino acid repeat comprises more than 52 glutamine residues. Thus, the nucleotide sequence of the mutant gene that codes for the mutant ATXN3 protein, comprises a trinucleotide repeat having more than 52 consecutive CAG codons in a coding exon. The wild-type or non-pathogenic form of ATXN3 comprises an amino acid repeat having no more than 52 glutamine residues, and consequently the nucleotide sequence of the gene coding for the wild-type or non-pathogenic form of ATXN3 comprises a trinucleotide repeat having no more than 52 consecutive CAG codons.

In another preferred embodiment of the method of the invention, the developmental transcription factor that comprises an aminoacid repeat mutation is CACNA1A, preferably wherein the amino acid repeat comprises more than 20 glutamine residues. Thus, the nucleotide sequence of the mutant gene that codes for the mutant CACNA1A protein, comprises a trinucleotide repeat having more than 20 consecutive CAG codons in a coding exon. The wild-type or non-pathogenic form of CACNA1A comprises an amino acid repeat having no more than 20 glutamine residues, and consequently the nucleotide sequence of the gene coding for the wild-type or non-pathogenic form of CACNA1A comprises a trinucleotide repeat having no more than 20 consecutive CAG codons.

In another preferred embodiment of the method of the invention, the developmental transcription factor that comprises an aminoacid repeat mutation is ATXN7, preferably wherein the amino acid repeat comprises more than 36 glutamine residues. Thus, the nucleotide sequence of the mutant gene that codes for the mutant ATXN7 protein, comprises a trinucleotide repeat having more than 36 consecutive CAG codons in a coding exon. The wild-type or non-pathogenic form of ATXN7 comprises an amino acid repeat having no more than 36 glutamine residues, and consequently the nucleotide sequence of the gene coding for the wild-type or non-pathogenic form of ATXN7 comprises a trinucleotide repeat having no more than 36 consecutive CAG codons.

In another preferred embodiment of the method of the invention, the developmental transcription factor that comprises an aminoacid repeat mutation is ATXN8, preferably wherein the amino acid repeat comprises from 80 to 250 glutamine residues. Thus, the nucleotide sequence of the mutant gene that codes for the mutant ATXN8 protein, comprises a trinucleotide repeat having from 80 to 250 consecutive CAG codons in a coding exon. The wild-type or non-pathogenic form of ATXN8 comprises an amino acid repeat having less than 80 glutamine residues, and consequently the nucleotide sequence of the gene coding for the wild-type or non-pathogenic form of ATXN8 comprises a trinucleotide repeat having less than 80 consecutive CAG codons.

In another preferred embodiment of the method of the invention, the developmental transcription factor that comprises an aminoacid repeat mutation is TBP, preferably wherein the amino acid repeat comprises more than 43 glutamine residues. Thus, the nucleotide sequence of the mutant gene that codes for the mutant TBP protein, comprises a trinucleotide repeat having more than 43 consecutive CAG codons in a coding exon. The wild-type or non-pathogenic form of TBP comprises an amino acid repeat having no more than 43 glutamine residues, and consequently the nucleotide sequence of the gene coding for the wild-type or non-pathogenic form of TBP comprises a trinucleotide repeat having no more than 43 consecutive CAG codons.

In another preferred embodiment of the method of the invention, the developmental transcription factor that comprises an aminoacid repeat mutation is PABPN1, preferably wherein the amino acid repeat comprises from 11 to 17 alanine residues. Thus, the nucleotide sequence of the mutant gene that codes for the mutant PABPN1 protein, comprises a trinucleotide repeat having from 11 to 17 consecutive GCN codons in a coding exon. The wild-type or non-pathogenic form of PABPN1 comprises an amino acid repeat having less than 11 alanine residues, and consequently the nucleotide sequence of the gene coding for the wild-type or non-pathogenic form of PABPN1 comprises a trinucleotide repeat having less than 11 consecutive GCN codons.

In another preferred embodiment of the method of the invention, the developmental transcription factor that comprises an aminoacid repeat mutation is ARX, preferably wherein the amino acid repeat comprises from 20 to 23 alanine residues. Thus, the nucleotide sequence of the mutant gene that codes for the mutant ARX protein, comprises a trinucleotide repeat having from 20 to 23 consecutive GCN codons in a coding exon. The wild-type or non-pathogenic form of ARX comprises an amino acid repeat having less than 20 alanine residues, and consequently the nucleotide sequence of the gene coding for the wild-type or non-pathogenic form of ARX comprises a trinucleotide repeat having less than 20 consecutive GCN codons.

In another preferred embodiment of the method of the invention, the developmental transcription factor that comprises an aminoacid repeat mutation is FOXL2, preferably wherein the amino acid repeat comprises from 20 to 23 alanine residues. Thus, the nucleotide sequence of the mutant gene that codes for the mutant FOXL2 protein, comprises a trinucleotide repeat having from 20 to 23 consecutive GCN codons in a coding exon. The wild-type or non-pathogenic form of FOXL2 comprises an amino acid repeat having less than 20 alanine residues, and consequently the nucleotide sequence of the gene coding for the wild-type or non-pathogenic form of FOXL2 comprises a trinucleotide repeat having less than 20 consecutive GCN codons.

In another preferred embodiment of the method of the invention, the developmental transcription factor that comprises an aminoacid repeat mutation is RUNX2, preferably wherein the amino acid repeat comprises 27 alanine residues. Thus, the nucleotide sequence of the mutant gene that codes for the mutant RUNX2 protein, comprises a trinucleotide repeat having from 27 consecutive GCN codons in a coding exon. The wild-type or non-pathogenic form of RUNX2 comprises an amino acid repeat having less than 27 alanine residues, preferably having 17 alanine residues, and consequently the nucleotide sequence of the gene coding for the wild-type or non-pathogenic form of RUNX2 comprises a trinucleotide repeat having less than 27, preferably having 17, consecutive GCN codons.

In another preferred embodiment of the method of the invention, the developmental transcription factor that comprises an aminoacid repeat mutation is PHOX2B, preferably wherein the amino acid repeat comprises from 25 to 33 alanine residues. Thus, the nucleotide sequence of the mutant gene that codes for the mutant PHOX2B protein, comprises a trinucleotide repeat having from 25 to 33 consecutive GCN codons in a coding exon. The wild-type or non-pathogenic form of PHOX2B comprises an amino acid repeat having less than 25 alanine residues, and consequently the nucleotide sequence of the gene coding for the wild-type or non-pathogenic form of PHOX2B comprises a trinucleotide repeat having less than 25 consecutive GCN codons.

In another preferred embodiment of the method of the invention, the developmental transcription factor that comprises an aminoacid repeat mutation is HOXA13, preferably wherein the amino acid repeat comprises from 22 to 29 alanine residues. Thus, the nucleotide sequence of the mutant gene that codes for the mutant HOXA13 protein, comprises a trinucleotide repeat having from 22 to 29 consecutive GCN codons in a coding exon. The wild-type or non-pathogenic form of HOXA13 comprises an amino acid repeat having less than 22 alanine residues, and consequently the nucleotide sequence of the gene coding for the wild-type or non-pathogenic form of HOXA13 comprises a trinucleotide repeat having less than 22 consecutive GCN codons.

In another preferred embodiment of the method of the invention, the developmental transcription factor that comprises an aminoacid repeat mutation is ZIC2, preferably wherein the amino acid repeat comprises 25 alanine residues. Thus, the nucleotide sequence of the mutant gene that codes for the mutant ZIC2 protein, comprises a trinucleotide repeat having from 25 consecutive GCN codons in a coding exon. The wild-type or non-pathogenic form of ZIC2 comprises an amino acid repeat having less than 25 alanine residues, preferably having 15 alanine residues, and consequently the nucleotide sequence of the gene coding for the wild-type or non-pathogenic form of ZIC2 comprises a trinucleotide repeat having less than 25, preferably having 15, consecutive GCN codons.

In another preferred embodiment of the method of the invention, the developmental transcription factor that comprises an aminoacid repeat mutation is HOXD13, preferably wherein the amino acid repeat comprises from 22 to 29 alanine residues. Thus, the nucleotide sequence of the mutant gene that codes for the mutant HOXD13 protein, comprises a trinucleotide repeat having from 22 to 29 consecutive GCN codons in a coding exon. The wild-type or non-pathogenic form of HOXD13 comprises an amino acid repeat having less than 22 alanine residues, and consequently the nucleotide sequence of the gene coding for the wild-type or non-pathogenic form of HOXD13 comprises a trinucleotide repeat having less than 22 consecutive GCN codons.

In another preferred embodiment of the method of the invention, the developmental transcription factor that comprises an aminoacid repeat mutation is ARX, preferably wherein the amino acid repeat comprises 20 alanine residues. Thus, the nucleotide sequence of the mutant gene that codes for the mutant ARX protein, comprises a trinucleotide repeat having from 20 consecutive GCN codons in a coding exon. The wild-type or non-pathogenic form of ARX comprises an amino acid repeat having less than 20 alanine residues, preferably having 12 alanine residues, and consequently the nucleotide sequence of the gene coding for the wild-type or non-pathogenic form of ARX comprises a trinucleotide repeat having less than 20, preferably having 12, consecutive GCN codons.

In another preferred embodiment of the method of the invention, the developmental transcription factor that comprises an aminoacid repeat mutation is SOX3, preferably wherein the amino acid repeat comprises 26 alanine residues. Thus, the nucleotide sequence of the mutant gene that codes for the mutant SOX3 protein, comprises a trinucleotide repeat having from 26 consecutive GCN codons in a coding exon. The wild-type or non-pathogenic form of SOX3 comprises an amino acid repeat having less than 26 alanine residues, preferably having 15 alanine residues, and consequently the nucleotide sequence of the gene coding for the wild-type or non-pathogenic form of SOX3 comprises a trinucleotide repeat having less than 26, preferably having 15, consecutive GCN codons.

In the present invention the term "nuclear magnetic resonance" or "NMR" refers to a spectroscopic technique which reports on changes on the chemical environment of nuclei, and therefore can be applied to characterize the structure of biomolecules. Being a time-based technique with atomic resolution, NMR has the unique ability to report on evolving signals belonging to nascent structures in a protein which appear with time, such as changes of the secondary structure (Alderson TR, Kay LE. NMR spectroscopy captures the essential role of dynamics in regulating biomolecular function. Cell. 2021 Feb 4;184(3):577-595). In the present invention, such incipient structures are coupled to irreversible liquid-solid transitions and represent the target for the screening of inhibitors or active agents.

As used in the present invention, the term "active agent" refers to any compound or composition that is able to modulate the structures responsible for the formation of aggregates of a transcription factor. The active agent may be selected from small molecules (molecules having a molecular weight ≤ 1000 daltons, also referred to as low-molecular weight molecules or compounds), proteins, peptides or nucleic acids. Preferably, the active agent is a chaperone protein or a fragment thereof.

In the present invention, the term "inhibitor of the changes in secondary structure that are responsible for the formation of aggregates" refers to an active agent that reduces or blocks changes in secondary structure that are responsible for the formation of aggregates.

The terms "reduce", "decrease" or "inhibit", used interchangeably in the present invention, are all used to mean a decrease by statistically significant amount. However, for avoidance of doubt, "reduced", "reduction" or "decrease" or "inhibit" means a decrease by at least 10% as compared to a reference level, for example a decrease by at least about 20%, or at least about 30%, or at least about 40%, or at least about 50%, or at least about 60%, or at least about 70%, or at least about 80%, or at least about 90% or up to and including a 100% decrease (i.e. absent level as compared to a reference sample), or any decrease between 10-100% as compared to a reference level.

In the method or use of the invention, the transcription factor that comprises an amino acid repeat mutation, or a fragment thereof, may be synthetised by chemical methods or using recombinant technology, wherein the recombinant technology comprises introducing a nucleic acid sequence that codes for the transcription factor or the fragment thereof, in host cell for its expression. The host cell may be eukaryotic, such as, but not limited to, *Saccharomyces cerevisiae*, or prokaryotic, such as, but not limited to, *Escherichia coli.*

In the NMR technique, the molecule or molecules to be analysed may be tagged with different isotopes such as $^1H$, $^2H$, $^{13}C$, $^{15}N$, $^{17}O$, $^{19}F$ or $^{32}P$. Thus, when the transcription factor is synthesised for its analysis by NMR, the aforementioned isotopes can be incorporated into the resulting molecule. In chemical synthesis, the aminoacids used may comprise any of the aforementioned isotopes. Or in the case of the synthesis with a recombinant host cell, the isotopes may be added to the culture media, for example, as being part of the molecules that conform the carbon source (e.g. $^{13}C$-glucose) or nitrogen source (e.g. $^{15}NH_4Cl$), so that the host cell uses these molecules for its metabolism and therefore incorporates them into the recombinant protein or peptide that is expressed.

In a preferred embodiment of the method of the invention, the transcription factor comprises $^1H$, $^{13}C$ and/or $^{15}N$.

The screening process is based on the observable differences of the NMR spectra of the isotopically labelled target molecule alone and that generated from the isotopically labelled target molecule in the presence of the active agent. When the target molecule is labelled in $^{15}N$ and $^{13}C$, two-dimensional $^{15}N/^1H$ or $^{13}C/^{15}N$ or $^{13}C/^{13}C$ correlation spectra is acquired on: i) the target (a transcription factor, or a fragment thereof, comprising an amino acid repeat mutation) molecule alone before and after incubation, ii) the target molecule (a transcription factor, or a fragment thereof, comprising an amino acid repeat mutation) in presence of the active agent before and after incubation. Then all spectra are compared to determine differences which are correlated to the effect of the active agent blocking incipient alternative structures in the target molecule. In particular, two-dimensional $^{15}N$-HSQC, $^{15}N$-HMQC, CON and CACO NMR spectra will be used, in addition to three-dimensional HNCA, CBCA(CO)NH and HNCACB spectra to identify structural differences in the target molecule in presence of the active compound.

Two-dimensional correlation spectra may also be used for correlating aminoacid repeat mutations and changes in secondary structure level responsible for the formation of aggregates of the transcription factor. When the transcription factor is labelled in $^{15}N$ and $^{13}C$, two-dimensional $^{15}N/^1H$ or $^{13}C/^{15}N$ or $^{13}C/^{13}C$ correlation spectra is acquired on: i) a wild-type or non-pathogenic transcription factor, or a fragment thereof, before and after incubation, and ii) a transcription factor, or a fragment thereof, comprising an amino acid repeat mutation, before and after incubation. Then the spectra are compared to determine differences which are correlated to the formation of aggregates. After the measurements i) and ii) the formation of aggregates is determined by any of the techniques previously defined, then allowing for the correlation between differences in NMR spectra representing changes in secondary structure and the formation of aggregates.

The electromagnetic radiations in the NMR are in the radio frequency region. So, in a preferred embodiment of the method of the invention the electromagnetic radiation is from 4 to 1200 MHz, preferably the radio frequency is 600 or 800 MHz.

In the present invention, the term "incubation" refers to a process by which a transcription factor, or a fragment thereof, comprising or not an amino acid repeat mutation, is maintained in environment for a period of time at an optimal temperature, enabling the transcription factor, or fragment thereof, to keep its structure (when the transcription factor is a wild-type or non-pathogenic transcription factor) or enabling the changes of its structural conformation (when the transcription factor comprises an aminoacid repeat mutation). The incubation may be performed in the presence or absence of an active agent, and when it is performed in the presence of an active agent it allows to assess the influence of said bioactive agent on the structure of the transcription factor. Terms "incubation" and "incubating" may be used interchangeably in the present invention.

The incubation may be carried out for at least 2h, preferably the incubation is carried out for a time period ranging from 2h to 168 h, more preferably form 24h to 96h. The temperature for the incubation is from 5ºC to 37 ºC. The transcription factor may be dissolved in a water solution, preferably a water-based buffer solution. The incubation buffer will preferentially be the NMR experimental buffer.

In another preferred embodiment of the method of the invention, the NMR measurements are carried out at a temperature from 5ºC to 37ºC, employing water-based buffer solutions such as, but not limited to, HEPES, PIPES, MES, sodium phosphate and potassium phosphate, with pH ranging from 6 to 8; and preferably having acontent of NaCl or KCl from 10 to 150 mM.

The present invention also refers to the use of a developmental transcription factor that comprises an amino acid repeat mutation, or a fragment thereof, for the *in vitro* screening by NMR spectroscopy of an active agent that inhibits changes in secondary structure in the transcription factor, wherein the changes in secondary structure are responsible for the formation of aggregates of said transcription factor, hereinafter the "use of the invention".

The terms used to define the present aspect referring to the use of the invention, have been explained in the preceding aspect referring to the method of the invention, and both they and the preferred embodiments thereof are applicable to the present aspect of the invention.

In another preferred embodiment of the use of the invention, the formation of protein aggregates is determined by light scattering, optical microscopy, electron microscopy, atomic force microscopy, analytical ultracentrifugation, chromatography or mass spectrometry.

In another preferred embodiment of the use of the invention, wherein the transcription factor comprises an amino acid expansion of a hydrophobic aliphatic amino acid or a hydrophilic polar uncharged amino acid; preferably wherein the hydrophobic aliphatic amino acid is alanine and wherein the hydrophilic polar uncharged amino acid is glutamine.

In another preferred embodiment of the use of the invention, wherein the amino acid expansion comprises at least 10 consecutive identical residues.

In another preferred embodiment of the use of the invention, wherein the amino acid expansion comprises from 10 to 250 consecutive identical residues.

In another preferred embodiment of the use of the invention, the transcription factor is a developmental transcription factor.

In another preferred embodiment of the use of the invention, the developmental transcription factor is selected form the list consisting of ATN1, HTT, JPH3-AS, AR, ATXN1, ATXN2, ATXN3 CACNA1A, ATXN7, ATXN8, TBP, PABPN1, ARX, FOXL2, RUNX2, PHOX2B, HOXA13, ZIC2, HOXD13, ARX and SOX3.

## DESCRIPTION OF THE DRAWINGS

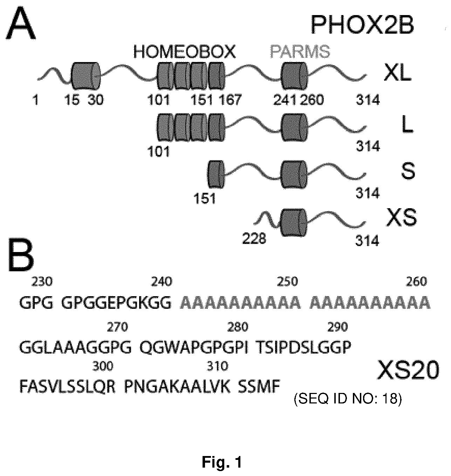

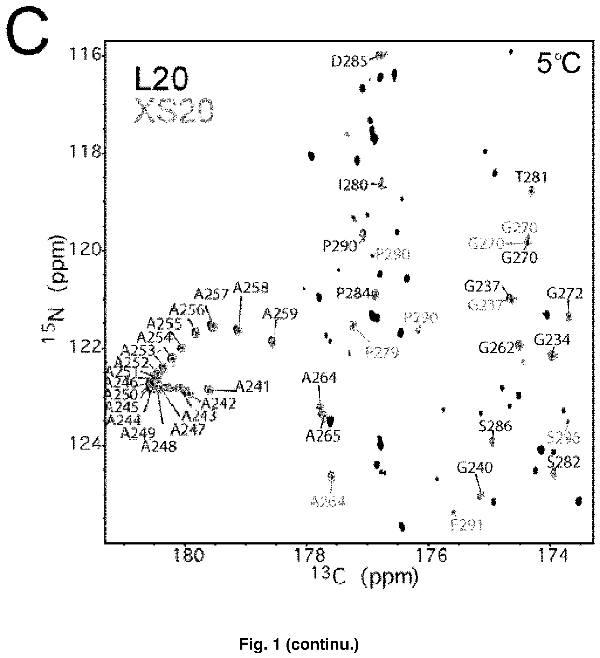

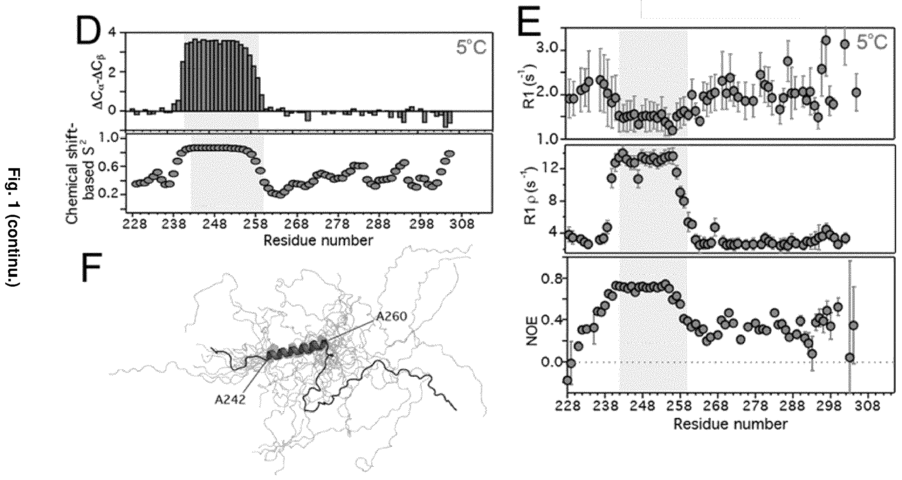

**Figure 1. PHOX2B polyalanine forms a stable a-helix in solution.** A) Different constructs containing PHOX2B domains. $\alpha$-helical regions are represented by cylinders. PARMS are located in the polyalanine (polyAla) segment covering residues 241-260. **B)** XS20 primary structure (SEQ ID NO: 18). The polyAla is highlighted in bold. **C)** Overlaid CON NMR spectra for L20 (black) and XS20 (grey) at 5°C. Assignments for XS20 are shown. Grey assignments correspond to alternative isomers. **D)** Secondary chemical shifts (top) and chemical shift-based $S^2$ order parameters (bottom) for XS20 at 5°C. **E)** $^{15}N$ spin relaxation parameters for XS20 at 5°C. **F)** Structural ensemble of the 20 XS20 conformers with the lowest conformational energy at 5°C. Structures were aligned onto the structured elements. The boundaries of the $\alpha$-helix (in grey) are indicated. The grey shadows in the plots in (D) and (E) limit the polyAla region. Protein concentration was 0.5 mM.

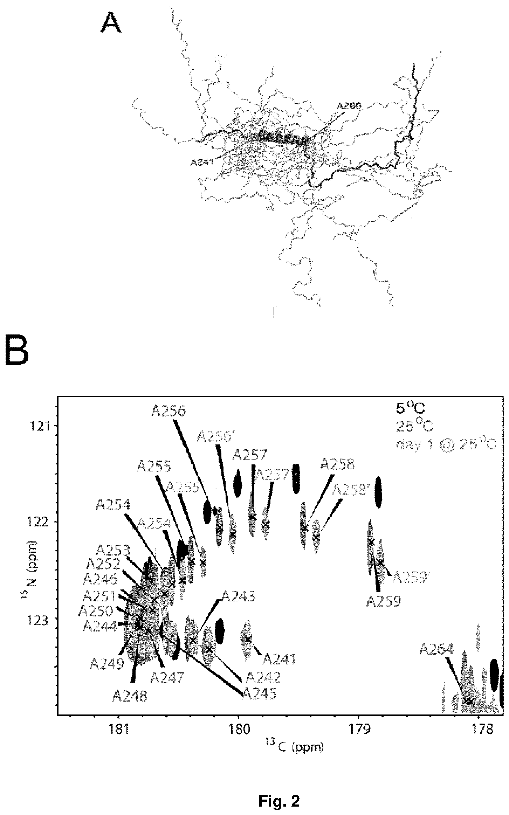

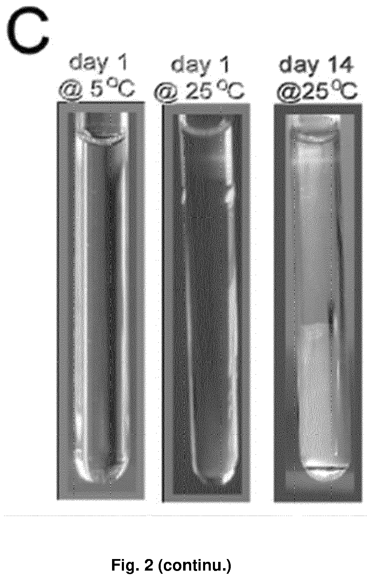

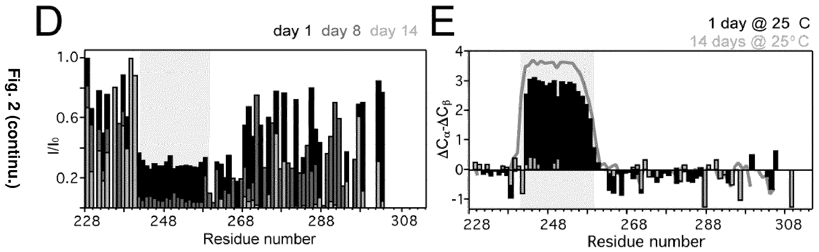

**Figure 2. PHOX2B $\alpha$-helical polyAla is coupled to temperature-dependent phase transitions. A)** Structural ensemble of XS20 20 lowest energy conformers at 25°C. The lowest energy structure is displayed on top of the ensemble. Boundaries of the $\alpha$-helix (in grey) are indicated. **B)** Overlaid CON spectra for XS20 $\alpha$-helical region at 5°C (black), 25°C (dark grey) and after 1 day at 25°C (light grey). Alternative conformations appearing upon incubation are labeled in light grey. This region disappears with longer incubation times. **C)** XS20 NMR samples following the indicated incubations. **D)** Decay in intensity plot for XS20 at day 1 (black), 8 (dark grey) and 14 (light grey) of incubation at 25°C. **E)** Secondary chemical shift plots for XS20 at 5°C (grey line), and after 1 day (black bars) and 14 days (light grey bars) of incubation at 25°C. Grey shadows in (**D, E**) limit the $\alpha$-helix. Protein concentration was 0.5 mM.

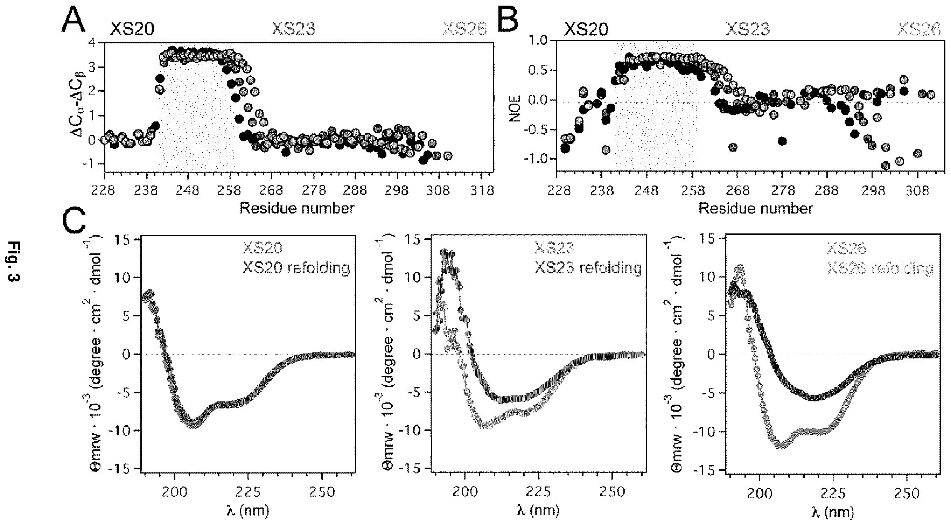

**Figure 3. PARMS extend the $\alpha$-helix promoting irreversible unfolding. A)** Secondary chemical shifts for XS20 (black), XS23 (dark grey) and XS26 (light grey) at 25°C. **B)** $^{15}N$ heteronuclear NOEs for XS20 (black), XS23 (dark grey) and XS26 (light grey) at 25°C. Errors bars (< 0.4) are not included for simplicity. The grey shadows in the plots in (**A-B**) limit the $\alpha$-helix in XS20. **C)** CD spectra for XS20 (light grey), XS23 (light grey) and XS26 (light grey) before and after heat denaturing (darker spectra in the plots). Protein concentration was 0.5 mM (**A-B**) and 0.17 mM (for XS23) and 0.36 mM (for XS20 and XS26) in (**C**).

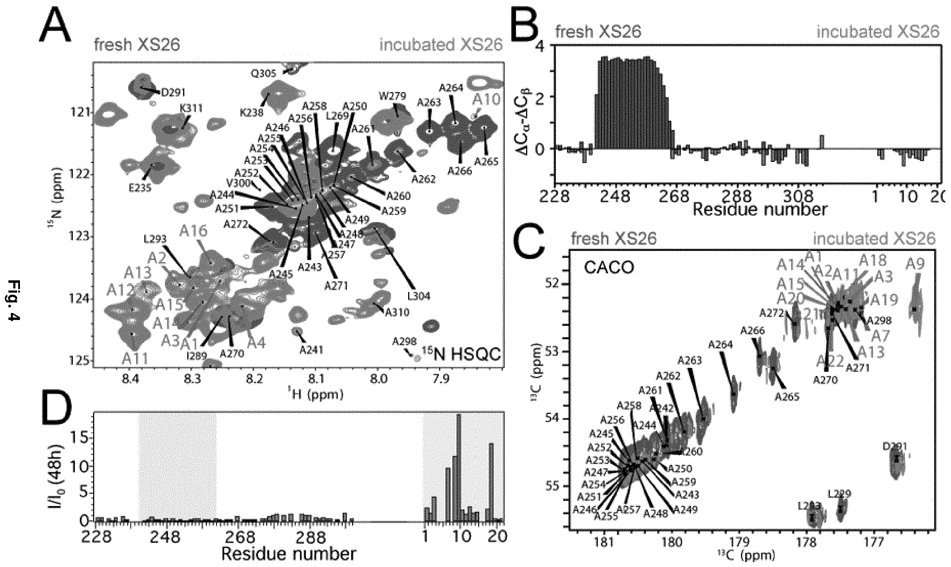

**Figure 4. PARMs promote nascent conformations. A)** Detailed $^{15}N$-HSQC spectra at 25°C for fresh XS26 (dark grey) and XS26 incubated 7 days at 25°C (light grey). **B)** Secondary chemical shifts for fresh XS26 (dark grey) and XS26 incubated 7 days at 25°C (light grey). Values for new alanine moieties are plotted on the right. **C)** Detailed CACO spectra at 25°C for fresh XS26 (dark grey) and XS26 incubated for 2 days at 25°C (light grey). **D)** CACO signal intensity for XS26 after 2 days at 25°C. Grey shadow limits XS26 polyAla $\alpha$-helix, while grey shadow on the right of the plot limits nascent disordered alanine moieties. Nascent alanine moieties (light grey in **A, C**) follow identical numbering and are plotted correlatively in (**B, D**). Protein concentration was 0.5 mM in (**A-B**) and 0.225 mM in (**C-D**).

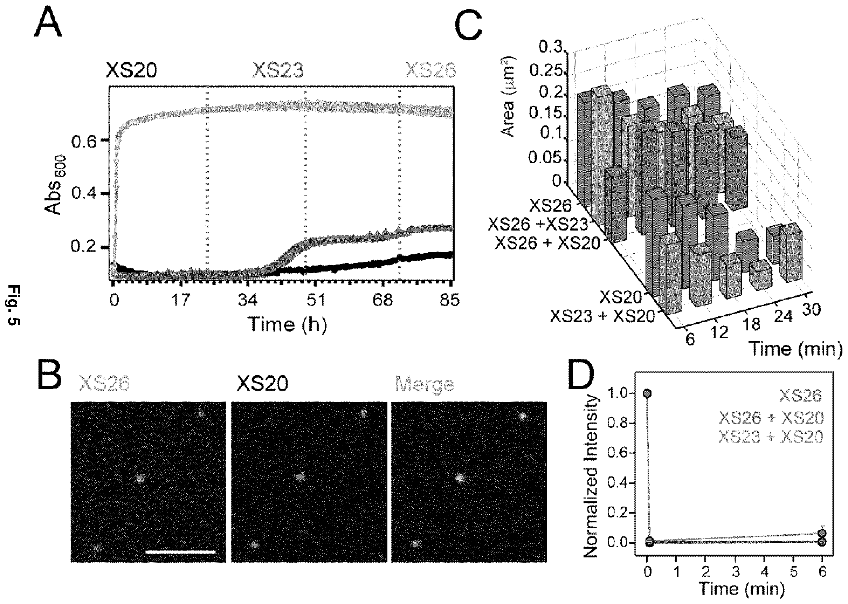

**Figure 5. Pathogenic PARMs promote rapid liquid-solid transitions. A)** Turbidity at 25°C for 0.225 mM XS20 (black), XS23 (dark grey) and XS26 (light grey). Broken lines represent consecutive days of incubation. **B)** Confocal fluorescent microscopy images showing colocalization of XS20 in XS26 condensates. XS26 was labeled with ATTO-488 and XS20 with ATTO-565. Scale bar: 5 $\mu$m. **C)** Histogram distribution of the area (in $\mu m^2$) of the

condensates formed by the protein mixtures with respect to the time spent for the microscopic observations. Error bars (<0.3 in $\mu m^2$) are not included for clarity. **D)** TIRF-FRAP data for XS26 condensates, and for mixtures of XS26+XS20 and XS23+XS20 incubated for 6-18 minutes at 25°C. Condensates showed marginal fluidity in all conditions tested and no fluorescence was recovered after bleaching. $n_{XS26}= 64$; $n_{XS26+XS20}= 50$; $n_{XS23+XS20}= 16$. Samples in (**B-D**) contained 0.1 mM XS23 and XS26 and 0.15-0.25 mM XS20.

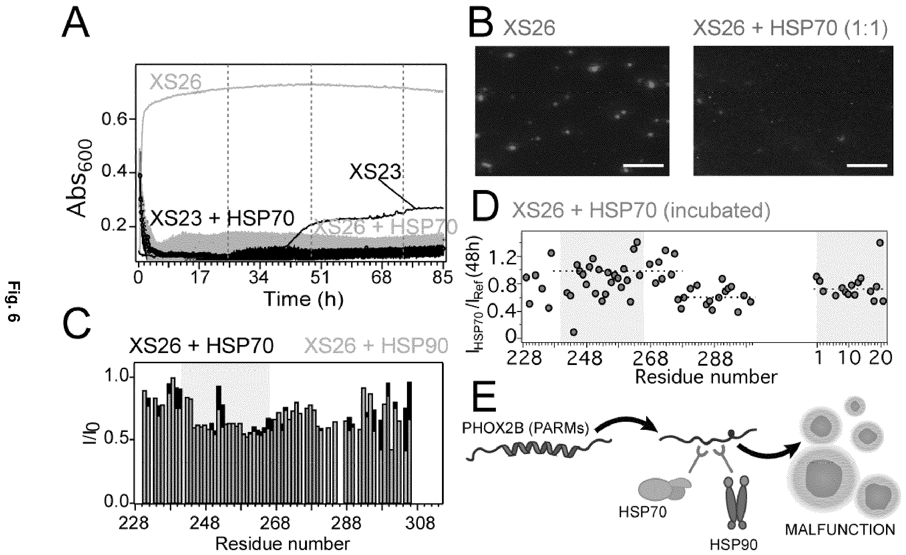

**Figure 6. Chaperones suppress PHOX2B de-mixing arresting nascent conformations. A)** Turbidity at 25°C for 0.225 mM XS23 or XS26 in presence of 0.1 mM HSP70 (black and light grey, respectively). Plots for the unaccompanied PHOX2B constructs are included. **B)** Fluorescence microscopy images for XS26 (0.1 mM, labeled with ATTO-565) in the absence and presence of HSP70. Scale bars= 10 $\mu m$. **C)** $^{15}$N-HSQC-based signal intensity plots for XS26 major conformation (70 $\mu M$) in complex with HSP70 (black) and HSP90 (light grey), all in 1:2 molar ratios. Grey shadow limits polyAla $\alpha$-helix. **D)** XS26 (0.225 mM) CACO signal intensity decay upon incubation (2 days at 25°C) in presence of HSP70 (1:2 equivalents). Grey shadow on the left limits polyAla $\alpha$-helix, while grey shadow on the right end limits nascent disordered alanine moieties. Broken lines mark the average intensity for the different segments. **E)** PARMs promote disorder in PHOX2B polyAla segment triggering fast liquid to-solid phase transitions. Chaperones arrest nascent disordered conformations blocking phase separation.

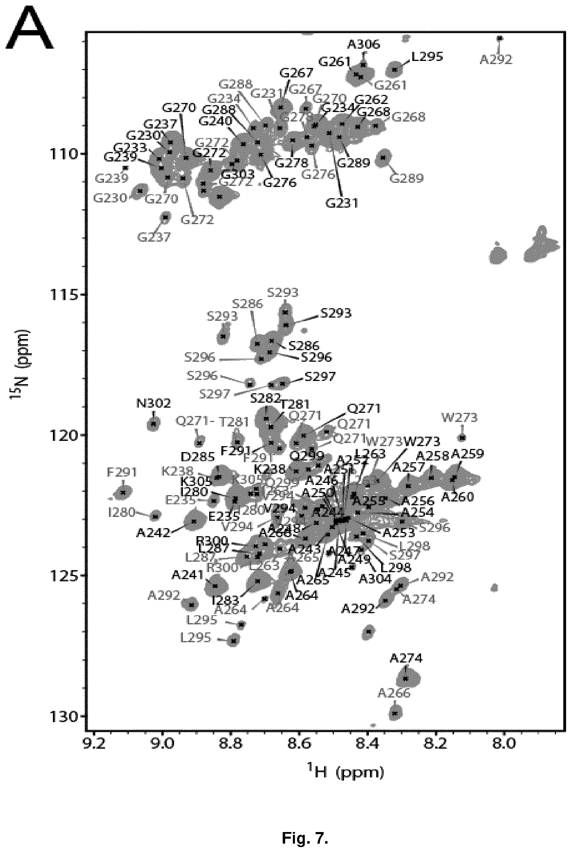

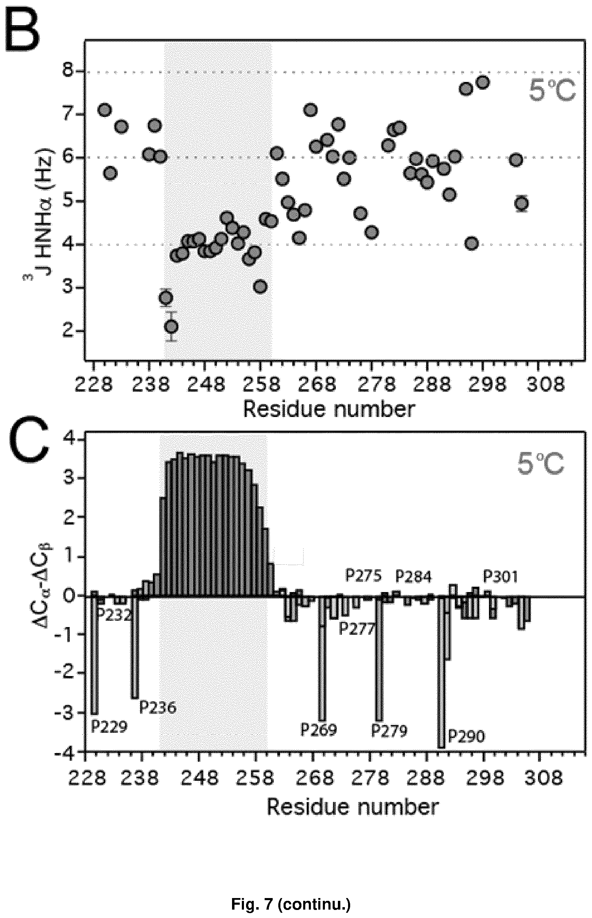

**Figure 7. XS20 assigned NMR spectra, J-couplings and alternative isomers. A)** Assigned $^{15}$N-HSQC spectrum of XS20 (0.5 mM) at 5°C. Alternative conformations are labeled in grey. **B)** J-coupling assessment of the secondary structure propensities for XS20 at 5°C. Values around 4 Hz indicate $\alpha$-helix formation, while values around 8 Hz are typical of $\beta$-strand structures. Errors are calculated based on NMR S/N ratio. **C)** Secondary chemical shifts for XS20 at 5°C. In these plots, positive values are representative of $\alpha$-helical conformations, while negative values correspond to $\beta$-strands. Prolines in *cis* isomer conformations (negative bars in the plot, light blue) amount to *circa* 15% and are responsible for the alternative conformations observed in the NMR spectra. The grey shadows in the plots in (**B**) and (**C**) limit the polyAla region.

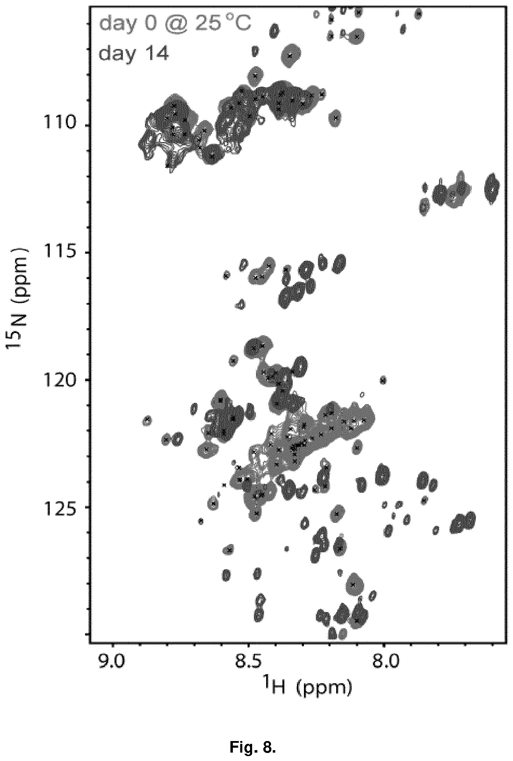

**Figure 8. $^{15}$N HSQC XS20 spectral changes upon incubation.** XS20 $^{15}$N-HSQC spectra at day 0 (light grey) and 14 (dark grey) of incubation at 25°C. The polyAla tract appears at 120-125 ($^{15}$N) and 8.1-8.4 ppm ($^1$H). Protein concentration was 0.5 mM.

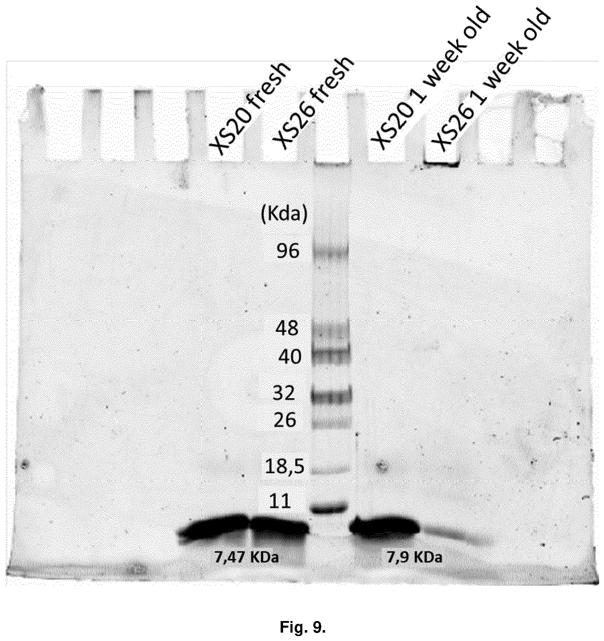

**Figure 9. SDS-PAGE of incubated samples shows no significant protein degradation.** 0.1 mM XS20 and XS26 were incubated for 7 days at 25°C showing no apparent degradation. Upon aggregation, a significant portion of incubated XS26 remains in the casted well unable to penetrate in the gel.

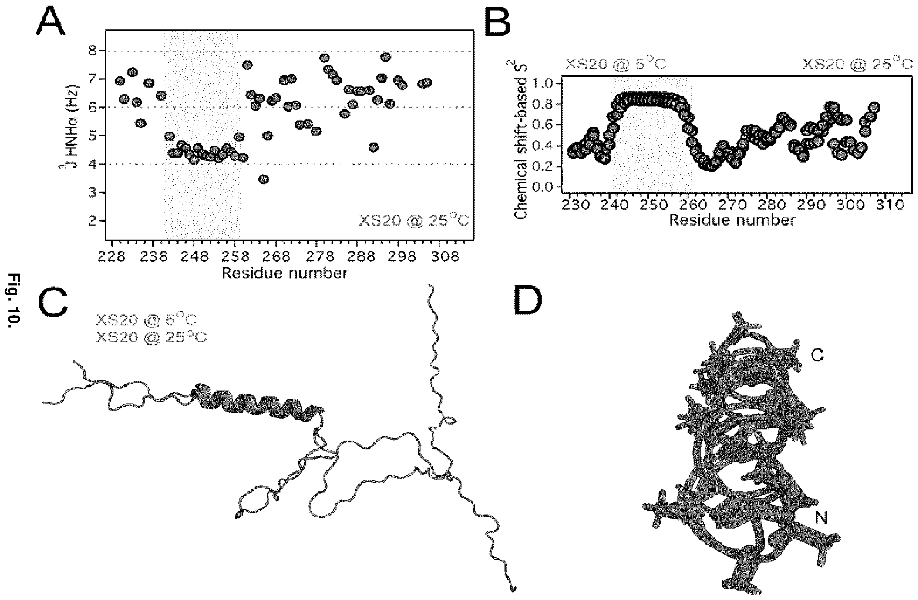

**Figure 10. XS20 J-couplings and S$^2$ order parameters at 25°C. A)** J-coupling assessment of the secondary structure propensities of XS20 at 25°C. **B)** Chemical shift-based S$^2$ order parameters calculated by TALOS-N for XS20 at 5°C (light grey) and 25°C (dark grey). **C)** Structural alignment of the lowest energy conformer of XS20 at 5°C (dark grey) and 25°C (light grey). **D)** Detail of the superimposed $\alpha$-helices of the polyAla tract from PHOX2B at 5°C (dark grey) and 25°C (light grey) showing that the sidechain disposition is barely identical. N- and C-terminus are indicated. The red shadows in the plots in (**A**) and (**B**) limit the $\alpha$-helix adopted by XS20 at 25°C.

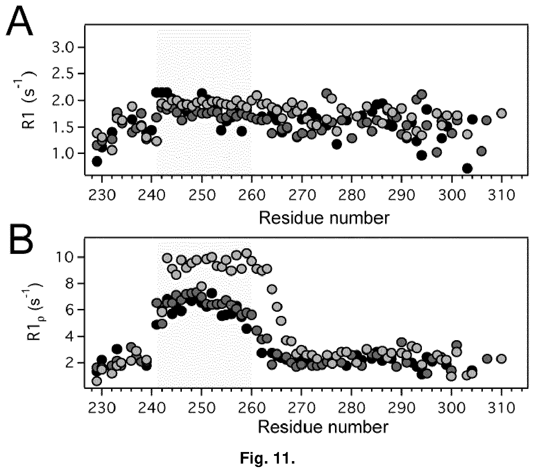

**Figure 11. PARMs promote intermolecular associations through the extended $\alpha$-helix. A-B)** $^{15}$N spin relaxation parameters for XS20 (black), XS23 (dark grey) and XS26 (light grey) at 25°C. Errors are lower than 1 s$^{-1}$ (R1, **A**) and 0.8 s$^{-1}$ (R1$\rho$, **B**) and error bars are not included for simplicity. The grey shadows in the plots limit the $\alpha$-helix in XS20.

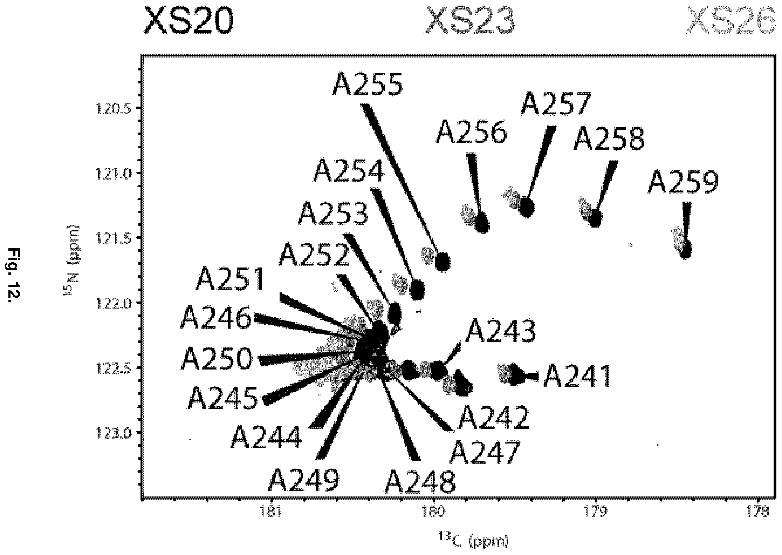

**Figure 12. The $\alpha$-helix from the polyAla region is preserved in the PARMs.** Shown is an overlay of the CON spectra for XS20 (black), XS23 (dark grey) and XS26 (light grey) at 25°C. The spectra are focused on the polyAla region. Sequence-specific assignments refer to XS20. Spectra are identically referenced. Protein concentration was 0.5 mM.

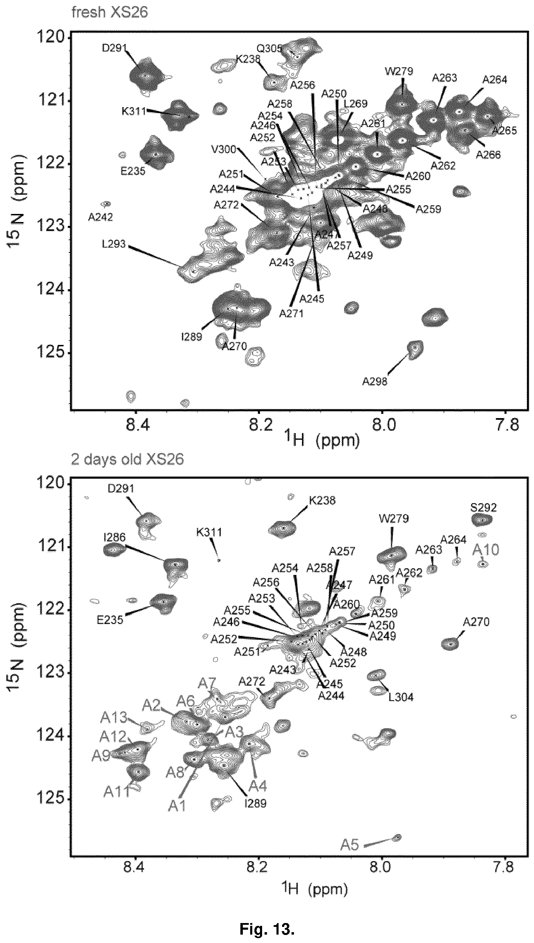

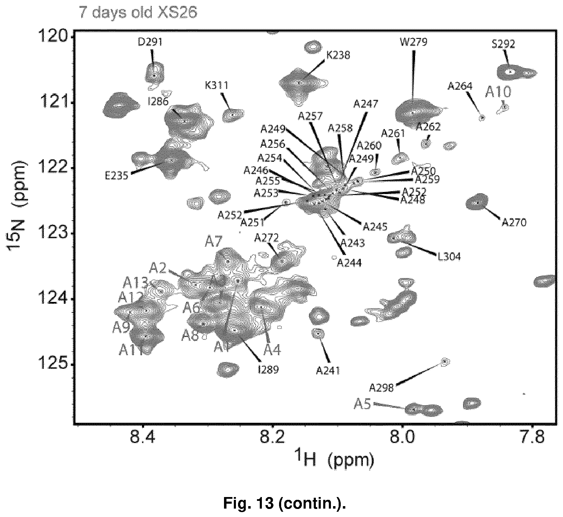

**Figure 13. The alternative polyAla conformations are reproducible and time-dependent.** Detailed $^{15}$N HSQC spectra from different XS26 samples (0.5 mM protein concentration) incubated at 25°C for different times showing that the new Ala moieties (labeled in grey) are reproducible and increase with the incubation time. The intensity of the $\alpha$-helix polyAla crosspeaks decrease with incubation time. The new Ala crosspeaks are numbered arbitrarily. Only nascent Ala crosspeaks are labeled, assignments of additional crosspeaks that appear upon incubation are not labeled for simplicity. Contour levels were increased on the spectra at the bottom (7 days old) to show the remaining signal intensity of the $\alpha$-helical polyAla crosspeaks.

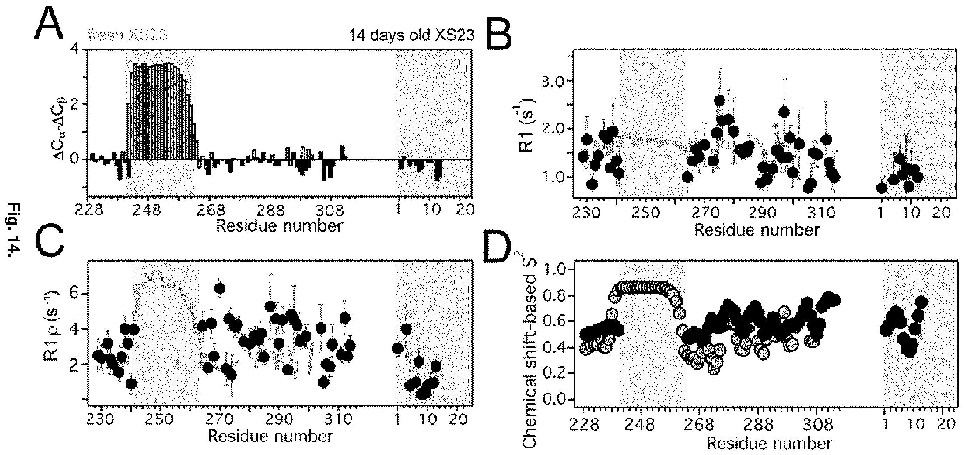

**Figure 14. PARMs adopt highly dynamic alternative conformations upon incubation. A)** Secondary chemical shifts for fresh XS23 (light grey bars) and for XS23 incubated for 14 days at 25°C (black bars). **B-C)** $^{15}N$ spin longitudinal ($R_1$, **B**) and rotating frame ($R_{1\rho}$, **C**) relaxation rates for fresh XS23 (grey line) and XS23 incubated for 14 days at 25°C (black markers). Error bars are not included in the orange lines for clarity. **D)** Chemical shift-based $S^2$ order parameters calculated by TALOS-N for fresh XS23 (grey markers) and XS23 incubated for 14 days at 25°C (black markers). The new alanine moieties appearing upon incubation at 25°C are located on the right of the plots and display disordered conformations. Grey shadows at the left of the plots limit polyAla α-helix, while grey shadows at the right side of the plots limit nascent disordered alanine moieties. Protein concentration was 0.5 mM.

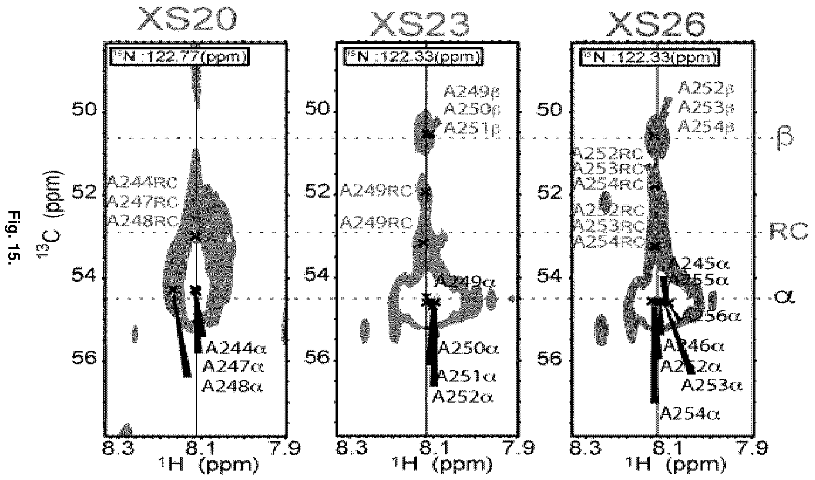

**Figure 15. PARMs promote structural conversions.** Strips from the 3D CBCA(CO)NH spectra at 25°C for XS20 (left), XS23 (centre) and XS26 (right) centered in the Calpha positions. The chemical shifts for the $^{15}N$-$^{1}H$ dimensions correspond to alanine resonances belonging to the central segment of the polyAla tract (**Fig. 4A**). Crosspeaks with chemical shifts typical for α-helical conformations are labeled α (black), RC for disordered (for random coil, grey), and β for β-strand conformations (grey). Protein concentration was 0.5 mM.

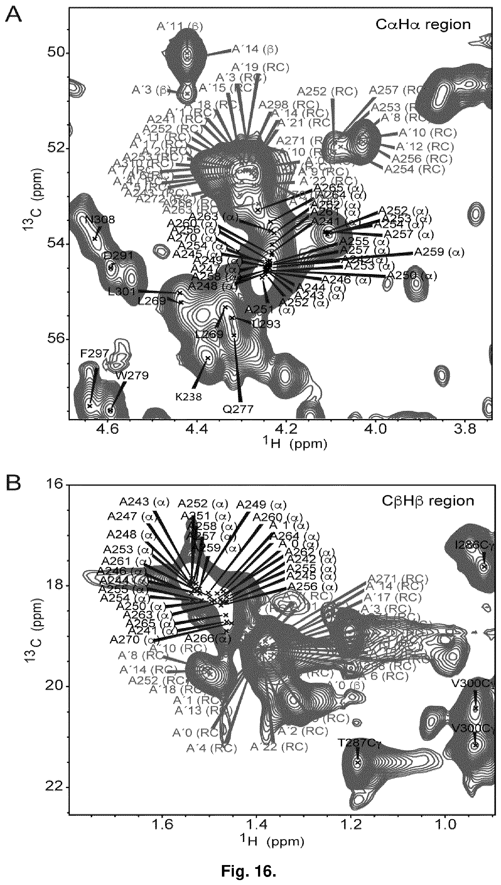

**Figure 16**. 13C-HSQC spectra reveal alternative polyAla conformations. A) Detail of the alanine moieties from the CαHα region of the $^{13}C$-HSQC spectrum. **B)** Detail of the alanine moieties from the CβHβ region. α-helical conformations are labeled in black (α), disordered conformations (random coil, RC) are labeled in grey, and β-strand conformations are labeled in grey (β). Sample was 0.5 mM XS26 after 24 h of incubation at 25°C. New alanine moieties are labeled with apostrophe and follow the numbering shown in Figure 13.

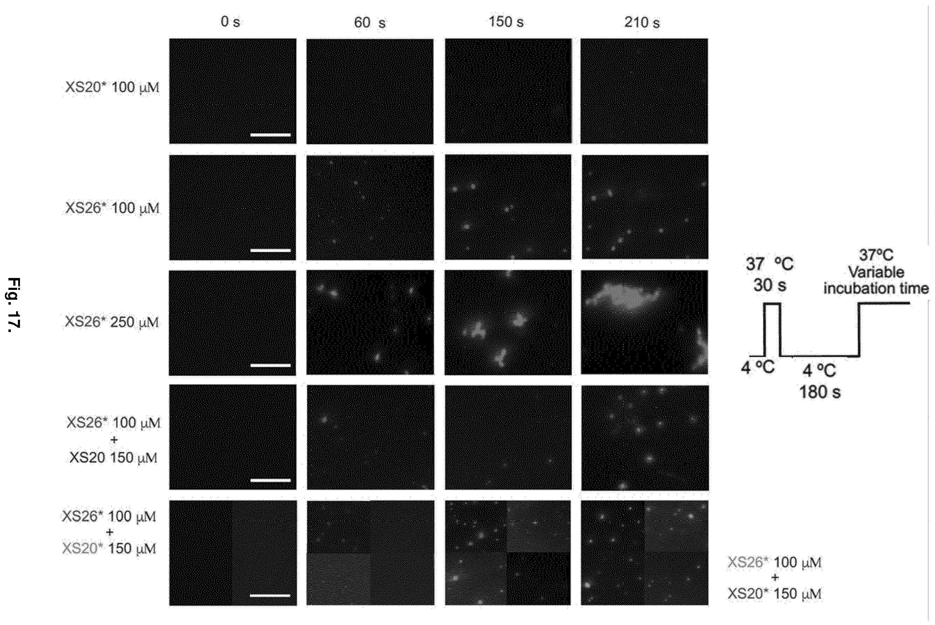

**Figure 17. XS26 triggers protein de-mixing and recruits XS20 into the condensates.** Fluorescent-labeled XS20 and XS26 (labeled with asterisk where required) were included at 1:400-600 ratios in the mixtures. XS26 at 0.25 mM shows fast amorphous aggregation. Images containing both labeled proteins (lower row) do not correspond to the same sample fields. XS20 is incorporated into the condensates at a slower rate. Incubation procedure is indicated on the right. Scale bars correspond to 10 μm.

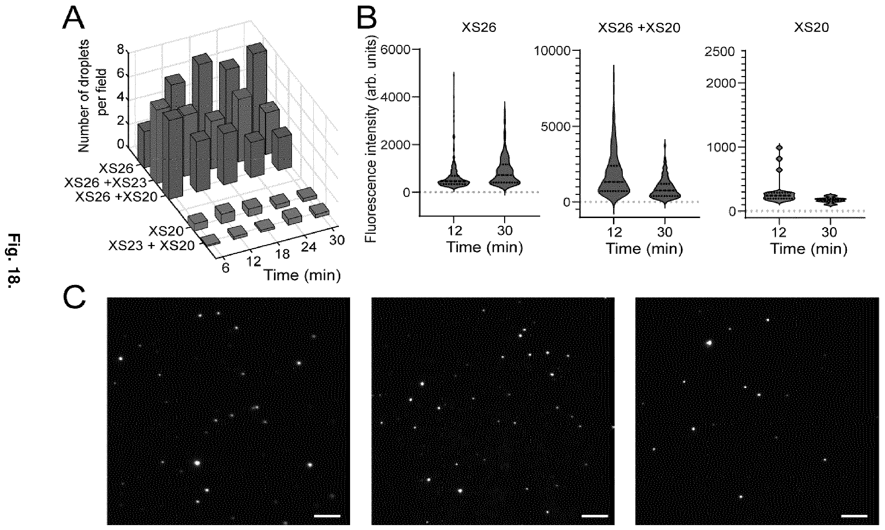

**Figure 18. Abundance and fluorescence intensity of the condensates. A)** Histogram distributions of the number of condensates formed by the different mixtures with respect to the time spent for the microscopic observations. The field was defined by the microscope setup and was limited by an area of 50,070*50,070 μm. n(XS26)= 1699; n(XS26+XS23)= 1247; n(XS26+XS20)= 1917; n(XS20)= 66; n(XS23+XS20)=66. **B)** Fluorescence intensity of the condensate formed by XS26 (left), XS26+XS20 (center) and XS20 (right) at 12 and 30 minutes of incubation. Protein concentration was 0.1 mM for XS26 and 0.15-0.25 mM for XS20. **C)** Representative images obtained by Nanoimager S microscope of the condensates formed by XS26 and included in the analyses. Scale bars= 5 μm.

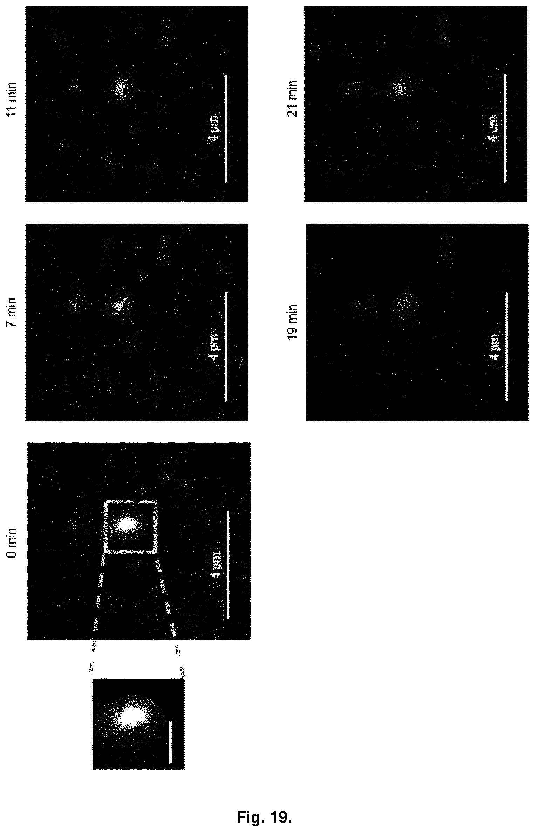

**Figure 19. Detail of a confocal-based FRAP experiment on a single condensate.** No fluorescence intensity was recovered after 21 minutes following photobleaching. This condensate corresponds to XS26 incubated at 0.1 mM. The scale bar on the insert at the left corresponds to 1 μm.

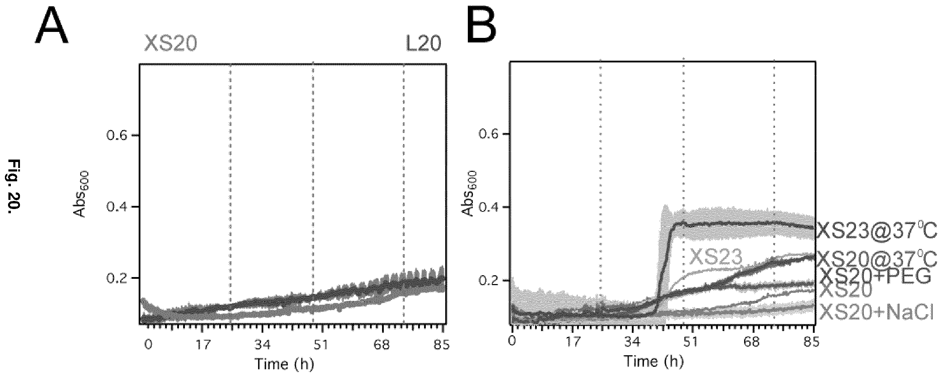

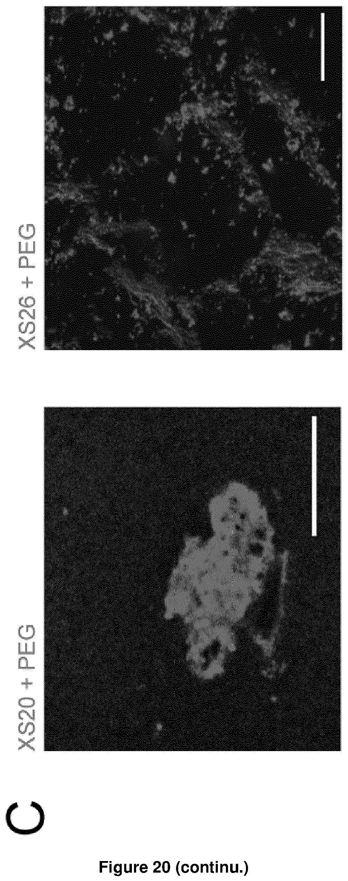

**Figure 20. Controls for PHOX2B phase transitions. A)** XS20 recapitulates longer PHOX2B construct phase transitions. XS20 is represented in light grey and L20 in dark grey. **B)** Addition of 10 % PEG (w/v) accelerates phase transition kinetics. Addition of 0.3 M NaCl has no effect on XS20 phase transitions (labelled as NaCl), indicating that hydrophobic aggregation is not the driving force underlying liquid-to-solid transitions. Incubation at 37°C accelerates phase transition kinetics both for XS20 and XS23, probably due to a temperature-dependent destabilization of polyAla α-helical conformation. XS20 and XS23 turbidity plots are included for comparison. **C)** Representative superresolution images of the amorphous aggregates formed by XS20 (left) and XS26 (right) with 10 % PEG (w/v) after 210 s of incubation following the protocol indicated in Figure 17. Scale bars= 10 μm. Proteins were labeled with ATTO-488. Protein concentration was 0.1 mM (**C**) and 0.225 mM (**A-B**) and incubation was performed at 25°C except where noted. Vertical broken lines represent the consecutive days of incubation (**A, B**).

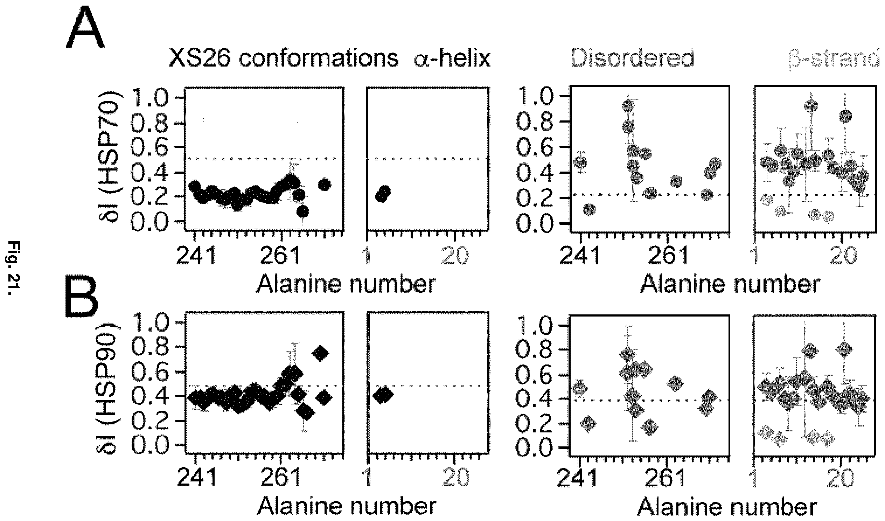

**Figure 21. Molecular chaperones target preferentially PHOX2B nascent conformations. A-B)** Decay in XS26 (0.1 mM) $^{13}C$-HSQC signal intensities upon addition of fresh HSP70 (**A**) and HSP90 (**B**, all in 1:1 molar ratios) for the different conformations of XS26 polyAla residues. Plots show α-helical (black), disordered (dark grey) and β-strand (light grey) polyAla conformations, and include averaged Cα and Cβ crosspeak intensities (Figure 16). X-axis are arranged based on polyAla residue numbering (241-266) or on the nascent Ala (1-23, in red on the axes). Broken lines in the plots represent the average of the signals contained in the corresponding pairing plot, for comparison.

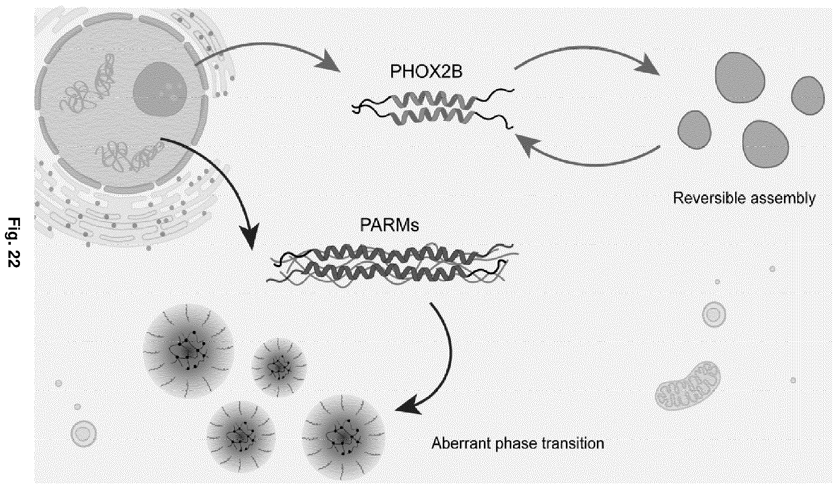

**Figure 22. Mechanistic model for the role of PARMs in PHOX2B pathogenicity.** PHOX2B establishes intermolecular interactions through the polyAla segment which could trigger reversible oligomer formation, both in the cytosol and in the nucleolus. PARMs induce metamorphism giving rise to disordered conformations which prime a fast liquid-to-solid transition. These solid condensates arrest wild-type PHOX2B, leading to PHOX2B loss-of-function and determining a dominant negative phenotype for CCHS.

**Examples**

**1. RESULTS**

**1.1 Results PHOX2B polyAla tract populates a rigid α-helix in solution**

The structural changes induced by polyalanine expansions (also called polyalanine repeat mutations or PARMs) in PHOX2B may alter PHOX2B's interactome and transcription function, leading to protein malfunction. We employed solution NMR spectroscopy to understand the structural impact of PARMs in PHOX2B. The polyAla tract present in the C-terminal domain of human PHOX2B is singularly conserved among mammals, ranging from 16 consecutive alanines in horse to 21 in cat. Sequence analysis and structural prediction reveal short structured elements flanked by long disordered regions in human PHOX2B (Fig. 1A-B). Several constructs of PHOX2B were generated containing different protein regions to enhance solubility and minimize spectral complexity (Fig. 1A). We focused on the fragment comprising the amino acid residues 228-314 (fragment termed XS20, for the number of consecutive alanines contained in the tract, SEQ ID NO: 18) of PHOX2B because its NMR spectra mirrored that of the longer PHOX2B fragments (Fig. 1C). Production of high-purity, highly soluble samples enabled us to acquire 3D NMR experiments on XS20 which follow backbone connectivities with high spectral resolution, circumventing the significant signal overlap from the polyAla repetitive region and enabling us to obtain unambiguous assignments (Fig. 1C, Fig. 7A).

Human PHOX2B transcription factor fragment comprising 20 alanine residues (XS20), SEQ ID NO: 18:

GPGGPGGEPGKGGAAAAAAAAAAAAAAAAAAAAGGLAAAGGPGQGWAPGPGPI
TSIPDSLGGPFASVLSSLQRPNGAKAALVKSSMF

Human PHOX2B transcription factor fragment comprising 23 alanine residues (XS23) SEQ ID NO: 19:

GPGGPGGEPGKGGAAAAAAAAAAAAAAAAAAAAAAAGGLAAAGGPGQGWAPGP
GPITSIPDSLGGPFASVLSSLQRPNGAKAALVKSSMF

Human PHOX2B transcription factor fragment comprising 26 alanine residues (XS26) SEQ ID NO: 20:

GPGGPGGEPGKGGAAAAAAAAAAAAAAAAAAAAAAAAAAGGLAAAGGPGQGWA
PGPGPITSIPDSLGGPFASVLSSLQRPNGAKAALVKSSMF

The polyAla region (residues 241-260, Fig. 1B) displayed a remarkable arrangement in the 2D CON NMR spectra (Fig 1C), where the consecutive alanine moieties mostly follow an N-to-C termini trail. NMR secondary chemical shift analysis and chemical shift-derived $S^2$ order parameters calculated by TALOS-N indicate that the polyAla tract particularly populates α-helical conformations, while the rest of the protein remains largely disordered (Fig. 1D). J-coupling analysis showed values close to 4Hz for the polyAla tract ( Fig. 7B), which is in agreement with a strong tendency to adopt α-helical structures Close inspection of the spectra reveals multiple minor conformations in the disordered regions due to significant cis-trans isomerization of the Pro residues in the numerous GPG motifs of PHOX2B (Fig. 1B, Fig. 7A,Fig. 7C). $^{15}$N spin relaxation experiments for XS20 at 5°C show a significant deviation of the longitudinal (R1) and rotating-frame (R1ρ) relaxation rates and the heteronuclear nuclear Overhauser effect (NOE) values for the polyAla region, indicating local rigidity due to the formation of secondary structure (Fig. 1E). The C-terminal region of the polyAla fragment appears less rigid, which would agree with the lower secondary chemical shift values observed (Fig. 1D). Overall correlation time ($\tau_C$) obtained from polyAla relaxation parameters (6,7 ns, Table 3) indicates that XS20 may establish intermolecular associations through the polyAla segment at 5°C.

A significant set of $^1$H-$^1$H NOE contacts were observed in $^1$H-$^{15}$N and $^1$H-$^{13}$C heteronuclear NOESY experiments which, in combination with chemical shifts and torsion angle restraints, defined distance restraints for XS20 structure calculation (Table 4). A set of 100 conformers were calculated by CYANA, and the 20 conformers with the lowest target function were selected and assessed by PROCHECKNMR (Fig. 1F). Almost the whole polyAla segment is contained in the α-helix (residues 242-260), which shows a significant structural convergence within all the conformers assessed (RMSD of 1.41 Å, Table 4). The rest of XS20 is highly dynamic, in close agreement with the chemical shifts, J-couplings and relaxation data (Fig. 1D, Fig. 1E, Fig. 7B).

## 1.2. PHOX2B undergoes a temperature-dependent de-mixing coupled to the polyAla α-helix

The solution structure of XS20 at 25°C was also calculated on the basis of experimental restraints (Fig. 2A, Table 4). The structure presents a well-delimited α-helix, covering the entire polyAla segment, flanked by highly disordered regions. Remarkably, the CON NMR spectra revealed the appearance of secondary conformations in XS20 samples upon incubation for 1 day at 25°C, particularly evident in the C-terminal region of the polyAla α-helix (Ala255-Ala259; Fig. 2B). These new conformations showed lower values in the CO dimension, indicative of decreased helical content. In addition, continued incubation of XS20 at 25°C promoted turbidity in the NMR samples (Fig. 2C). Analysis of the $^{15}$N HSQC NMR spectra for the incubated XS20 samples indicated significant signal decay in the polyAla region (Fig. 2D, Figure 8). Because an increase in turbidity is associated with protein de-mixing or aggregation processes, we wondered whether the NMR signal decay observed in the polyAla segment was due to intermolecular associations involved in PHOX2B assemblies or rather to structural conversions coupled to phase transitions. Analysis of the secondary chemical shifts indeed revealed that incubated XS20 showed a time-dependent decrease in the α-helical content of the polyAla region (Fig. 2E), which suggests that PHOX2B displays metamorphism towards disordered structures upon de-mixing or aggregation. Importantly, XS20 showed no significant degradation during long incubations at 25°C (Fig. 9). Therefore, increase in order parameters or protein degradation are not responsible for the signal decay detected in the polyAla region upon incubation (Fig. 2D).

J-coupling assessment and chemical shift-based order parameters confirmed that the structure of XS20 is largely identical at 5°C and 25°C (Fig. 10). Moreover, the $^{15}$N spin relaxation parameters for XS20 at 25°C indicate that the protein maintains the main rigid element covering the polyAla segment, with increased flexibility in the C-terminal region of the α-helix (Fig. 3A-B, Figure 11). $\tau_C$ calculations based on experimental relaxation data indicates that XS20 may also assemble through the polyAla tract at 25°C (Table 3). Considering that the major structures of XS20 at 5°C and 25°C are fairly identical (Fig. 10C, Fig 10D), the remarkable propensity of PHOX2B to self-associate at higher temperatures upon incubation could be attributed to the increased contact potential of hydrophobic Ala sidechains at 25°C or to temperature-dependent structural rearrangements.

## 1.3 PARMs extend the major α-helical conformation in the polyAla region

Pathogenic PARMs are expected to alter protein conformation impacting interactomes. To determine the effect of PARMs on PHOX2B structure, we generated a +3 Ala variant (adding 3 alanine residues to the 20 polyAla tract, named XS23 SEQ ID NO: 19) and a +6 Ala variant (named XS26, SEQ ID NO: 20). While a +3 Ala expansion would not be strongly correlated to CCHS, XS26 would be representative of the most abundant PARMs observed in the disease (Di Lascio, S. et al. Hum Mutat 39, 219-236 (2018)).. NMR secondary chemical shifts showed that the Ala inclusions in XS23 and XS26 extend the α-helix covering the polyAla segment (Fig. 3A). In addition, $^{15}$N spin relaxation data indicated that the expanded α-helices from XS23 and XS26 are also rigid and show a stronger propensity to establish length-dependent intermolecular associations at 25°C, particularly evident for XS26 (Fig. 3B, Figure 11, Table 3). Therefore, PARMs do not alter the structural and dynamic properties of the major α-helical conformation of the polyAla tract but increase its self-assembly potential.

At low temperatures, polyAla repeats are assumed to form coiled-coil structures that increase in stability with polyAla length (Pelassa, I. et al. Hum Mol Genet 23, 3402-20 (2014)). However, PARMs were also reported to decrease PHOX2B thermal stability favoring amyloid aggregation (Pirone, L. et al. FEBS J 286, 2505-2521 (2019). Circular dichroism (CD) measurements determined that both XS20 and the expanded variants display a high content in α-helical structure (Fig. 3C). Spectral analysis revealed no indication of coiled-coil formation for any PHOX2B variant at 25°C. Strikingly, although XS20 showed a fully reversible refolding after heat unfolding, both XS23 and XS26 showed irreversible unfolding. Indeed, the spectra revealed an α-to-β switch similar to that reported elsewhere for designed peptides(Ciani, B., Hutchinson, E.G., Sessions, R.B. & Woolfson, D.N. J Biol Chem 277, 10150-5 (2002)). Therefore, we wondered whether PARMs modulate the equilibrium between conformational states giving rise to nascent conformations which could promote associations into higher order species.

## 1.4. PARMS promote alternative minor conformations

The 2D CON spectra showed shifts towards higher values in the CO dimension for the expanded polyAla fragments, which is indicative of a stronger tendency to populate α-helical conformations (Fig. 12). However, incubation of XS26 and XS23 at 25°C promoted considerable sample turbidity, triggering significant changes in the $^{15}$N HSQC NMR

spectra (Fig. 4A). Besides the disappearance of the polyAla $\alpha$-helical region, as described for XS20 after prolonged incubations (Fig. 8), new crosspeaks appeared in the spectra which were not attributable to protein degradation (Fig. 9). Sequential assignments corroborated that the incipient crosspeaks belong to Ala residues which display disordered conformations, clearly identified by the characteristic chemical shifts for alanine's C$\alpha$ and C$\beta$ moieties (Fig. 4A-B). The signal intensity of these new Ala resonances increase with the incubation time, and is highly reproducible among different protein batches (Fig.13). $^{15}$N spin relaxation measurements for the incubated samples confirmed that the new polyAla crosspeaks are highly dynamic, in stark contrast with the major, rigid $\alpha$-helical conformation for the polyAla segment in fresh samples (**Fig.14, Table 3**).

In addition, comparison of the 3D NMR CBCA(CO)NH spectra for XS20, XS23 and XS26 showed that the region where central polyAla crosspeaks appear additionally contained emerging crosspeaks with chemical shifts which are characteristic for alanines in disordered (or random coil; 52,67 +/- 1,76 ppm) or $\beta$-strand (50,86 +/- 1,28 ppm) conformations (Fig.15). Remarkably, only the expanded XS23 and XS26 variants showed the nascent crosspeaks, with higher signal intensity for XS26. However, strong signal overlap in this region of the spectra could lead to dubious spectral processing. To discard misassignment due to artifactual spectral processing, the presence of the alternative conformations in the PARMs was validated in 2D NMR spectra with direct $^{13}$C detection (CACO spectra), in addition to 2D $^{13}$C HSQC spectra (Fig. 4C, Fig. 16). CACO spectra provided also direct evidence of the emergence of polyAla disordered conformations upon incubation (Fig. 4D). Therefore, PARMs promote alternative minor conformations in PHOX2B, which could be the basis for their reported aggregation propensity (Bachetti, T. et al. Hum Mol Genet 14, 1815-24 (2005); Di Zanni, E. et al. aggregates containing polyAla expanded PHOX2B proteins. Neurobiol Dis 45, 508-18 (2012);).. However, a direct correlation between the abundance of the alternative polyAla conformations in PHOX2B and its propensity to aggregate, is missing.

### 1.4. Pathogenic PARMS trigger rapid liquid-liquid and liquid-solid transitions

Light dispersion measurements showed a length-dependent increase in turbidity for PHOX2B, with a rapid response for XS26 (Fig. 5A). Confocal microscopy confirmed that XS26 promoted phase de-mixing rather than fibrillar aggregation (Fig. 5B). Interestingly, the condensates triggered by XS26 de-mixing incorporated XS20, which correlates with the dominant negative effect of the pathogenic PARMs in PHOX2B (Di Lascio, S. et al. Neurobiol Dis 50, 187-200 (2013)). Fluorescence microscopy validated the minimal de-mixing for XS20 (Fig. 17, Fig. 18A). In addition, XS20 is arrested into XS26 condensates at a slower rate (Figure 17), in agreement with the reported slower kinetics of aggregation for the shortest PHOX2B variants (Fig. 5A).

A recent study reported that PARMs in the protein HOXD13 alter its de-mixing properties and its capacity to co-condense with transcriptional co-activators, presenting dysregulated phase separation as the potential basis for human synpolydactyly (Basu, S. et al. Cell 181, 1062-1079.e30 (2020)). To determine the phase transition properties of XS26, we performed fluorescent microscopy experiments on individual condensates (Fig. 5C, D). Recruitment of XS20 into heterotypic condensates promoted a time-dependent reduction in condensate size and fluorescent intensity (Fig. 5C, Fig. 18B), which is indicative of lower protein content due to diffusion in the aged condensates containing XS20. The considerable small size of PHOX2B condensates (< 1 $\mu$m, Fig. 5B-C, Fig.17, Fig.18C) makes the acquisition of fluorescent recovery after photobleaching (FRAP) measurements by confocal microscopy highly challenging (Fig.19). Thus, FRAP measurements were performed on total internal reflection fluorescence (TIRF). TIRF-FRAP rates revealed minimal mobility inside the homotypic and heterotypic PHOX2B condensates (Fig. 5D), consistent with confocal-based FRAP measurements (Fig.19). Therefore, PARMS promote a rapid transition into solid condensates, which can retain wild type PHOX2B. Interestingly, PHOX2B phase transitions are not affected by increased ionic strength (Fig.20), suggesting that, while hydrophobicity may promote small oligomers via polyAla coiled coils, phase de-mixing is not driven by polyA's hydrophobicity. However, increasing temperatures accelerated phase transition kinetics (Figure 20B), which may be explained by enhanced thermal destabilization of polyAla major conformation. Therefore, we wondered whether the structural disorder induced by PARMs is coupled to phase transitions.

### 1.5 Molecular chaperones arrest PHOX2B nascent conformations suppressing phase transitions

Molecular chaperones are essential for neutralizing protein misfolding and malfunction. Heat shock proteins HSP70 and HSP40 co-localize with cellular PHOX2B aggregates, and are directly involved in their clearance (Trochet, D. et al. Hum Mol Genet 14, 3697-708 (2005)) Turbidimetry and fluorescence microscopy revealed that HSP70 minimized XS26 phase transitions (Fig. 6A, B). Because phase transitions are coupled to rearrangements within the polyAla $\alpha$-helix (Figs. 2-4), we reasoned that chaperones diminish PHOX2B homo-associations by binding the polyAla $\alpha$-helix in high affinity. This hypothesis would agree with the chaperone capacity to recognize hydrophobic, structured motifs in disordered clients. Surprisingly, NMR titrations showed that HSP70 and HSP90 molecular chaperones are not preferentially bound to PHOX2B polyAla $\alpha$-helix (Fig. 6C). However, since there is a patent correlation between the emergence of polyAla alternative conformations and the propensity to phase separate by PHOX2B (Fig. 4, Fig. 5), we considered that chaperones could target specifically polyAla alternative conformations rather than the major $\alpha$-helical structure. 2D $^{13}$C HSQC and CACO spectra provide convenient templates to confirm this specific recognition because they expose the whole diversity of conformations in the polyAla tract and are short time consuming. Moreover, because chaperone binding will result in signal broadening beyond detection, increased difference in signal intensity in presence of chaperones is an indication of preferential chaperone targeting. Analysis of signal intensity of the different polyAla conformations in XS26 revealed that HSP70 and HSP90 molecular chaperones predominantly target disordered conformations (Fig. 6D, Fig. 21). In brief, the signal intensity of XS26 polyAla tract in $\alpha$-helical conformation diminishes up to 20% in presence of freshly added HSP70 and 40% for HSP90, while the intensity of the polyAla moieties in disordered conformation are reduced above 50% in the presence of chaperones (Fig.21). In addition, the population of alternative disordered alanines that emerge upon incubation are selectively targeted by HSP70, and diminish about 50 % after 48 h of incubation (Fig. 6D). Therefore, the data clearly indicate that chaperones target disordered nascent conformation induced by PARMs, which are the template structures priming phase transitions (Fig. 6E).

## 2. DISCUSSION

About 15% of eukaryotic proteins contain homorepeats. Polyalanine, polyserine and polyglutamine segments primarily adopt $\alpha$-helical conformations, reportedly promoting coiled coil associations triggering length-dependent aggregation (Pelassa, I. et al. Genet 23, 3402-20 (2014)). Interestingly, polyAla coiled coils showed a higher tendency to aggregate compared to polyQ and polyS (Lilliu, E. et al. J Struct Biol 204, 572-584 (2018), explaining why polyAla homorepeats are usually shorter and less polymorphic than polyQ repeats and display lower thresholds for disease-causing TREs. PHOX2B contains the longest polyAla segment known in mammals and one of the shortest thresholds for disease-causing expansions (Amiel, J. et al. Nat Genet 33, 459-61 (2003);). Long PARMs in PHOX2B cause shifts in localization and cytoplasmic aggregation (Di Lascio, S. et al. Neurobiol Dis 50, 187-200 (2013).. Therefore, a loss-of-function mechanism triggered by aggregation has been postulated as the basis for mutated PHOX2B malfunction in CCHS (Di Lascio, S. et al. Neurobiol Dis 50, 187-200 (2013)). Similar mechanisms were proposed for the impact of PARMs in SOX3, RUNX2, FOXL2, HOXA13 and HOXD13. However, clear connections between expanded homorepeats aggregation and cellular toxicity are still missing. In addition, the nature of PARM aggregates remained controversial, since both amyloid and $\alpha$-helical-rich aggregates have been described for polyAla fragments. Most of the PARMs are related to the origin of human developmental diseases, which would be in agreement with PARM-induced protein chronic dysfunction, rather than with amyloid aggregation. Indeed, lack of detection of PHOX2B aggregates in mouse models (Dubreuil, V. et al. Proc Natl Acad Sci U S A 105, 1067-72 (2008) is consistent with the assumption that PARMs may cause toxicity triggering aberrant small oligomers. Frameshift mutations in PHOX2B promote nucleolar mispartitioning and cytotoxicity by aberrant phase transitions (Mensah, M.A. et al. Nature 614, 564-571 (2023)). In addition, PARMs in HOXD13 dysregulate LLPS, favoring homotypic phase separation and reducing condensate fluidity (Basu, S. et al. Unblending of Transcriptional Condensates in Human Repeat Expansion Disease. Cell 181, 1062-1079.e30 (2020)). This aberrant de-mixing lessens the blending of HOXD13 with transcription factors in heterotypic condensates, potentially inhibiting transactivation and ultimately promoting human synpolydactyly. Our data indicate that a similar mechanism could underly expanded PHOX2B loss-of-function in CCHS (Fig.22). Pathogenic PARMs in PHOX2B trigger fast liquid-to-solid transitions which seclude the wild-type form. Loss of PHOX2B function is therefore previous to aggregation into large protein deposits, consistent with the lack of protein aggregation in mice models of human CCHS. Still, PHOX2B condensates evolve into irregular clusters (Fig.17, Fig. **20C**) rather than fibrillar aggregates, explaining why expanded PHOX2B is deposited in the cell cytoplasm upon over-expression (Di Lascio, S. et al. Neurobiol Dis 50, 187-200 (2013)). These features are also present in other PARMs, in agreement with a common mechanism underlying polyAla human diseases (Basu, S. et al. Unblending of Transcriptional Condensates in Human Repeat Expansion Disease. Cell 181, 1062-1079.e30 (2020)). Interestingly, a recent report revealed cytosolic condensates triggered by polyS motifs which recruit and promote tau aggregation (Lester, E. et al. Proc Natl Acad Sci U S A 120, e2217759120 (2023)). Therefore, condensate formation could represent novel targets for therapeutic intervention in TRE human diseases, towards the discovery of compounds that modify the properties or prevent biomolecular condensates.

Basu and colleagues showed that condensate fluidity decreased dramatically with the length of the expanded PARM (Basu, S. et al. Cell 181, 1062-1079.e30 (2020)). In particular, 23-alanine HOXD13 showed minimal fluidity, and 25-alanine HOXA13 and 27-alanine RUNX2 showed absence of FRAP, indicating the solid properties of the aggregates formed. Here, we observed fast liquid-to-solid transitions in PHOX2B condensates promoted by a +6 alanine expansion (26 alanines), which are not alleviated by the recruitment of wt PHOX2B (20 alanines) into the condensates. Interestingly, the solid condensates formed by a +3 alanine expansion showed a remarkable delayed phase transition. Because a +3 PARM in PHOX2B is not significantly related with CCHS, we could postulate that fast liquid-to-solid transitions triggered by long PARMs would promote protein malfunction, and solid condensates should therefore be considered the relevant therapeutic targets. Similar to HOXD13 (Basu, S. et al. Cell 181, 1062-1079.e30 (2020)), malignancy of PHOX2B condensates could result from the absence of additional partners in heterotypic condensates due to the fast de-mixing of expanded PHOX2B. Indeed, because of its ability to directly detect the abundance of alternative conformations responsible for phase transitions, NMR spectroscopy is a useful screening platform for structure-based design of phase transitions inhibitors. Importantly, because imbalanced phase transitions could well be the basis for protein misfunction in PARMs, the structural approach presented here could be useful to target several human diseases. Yet, revealing the protein structures and interactions that drive aberrant phase transitions is indeed highly advantageous to design compounds that decrease the population of these active states.

Here, we report PHOX2B C-terminal atomic structures including the polyAla fragments, and reveal that PARMs alter the conformational transitions promoting nascent structures. Previous studies detected conformational changes induced by homorepeat expansions which could be modulated by the flanking regions and protonation states (Baias, M. et al. J Am Chem Soc 139, 1168-1176 (2017)). Indeed, flanking sequences modulate conformational transitions and aggregation propensities in homorepeats (Escobedo, A. et al. Nat Commun 10, 2034 (2019)). However, a direct observation of the co-existence of different conformations in expanded homorepeats in steady state was lacking. Taking advantage of the increased NMR signal intensity of the core of the polyAla tract moieties, in addition to the characteristic alanine chemical shifts, we observed the emergence of alternative conformations in PARMS coupled to phase transitions. In brief, we detected abundant conversions from $\alpha$-helical to disordered conformations for the polyAla upon phase transitions, in agreement with the structural conversions previously proposed for polyQ tracts and measurements of distant proteins in the dense phases (Burke, K.A., Janke, A.M., Rhine, C.L. & Fawzi, N.L. Mol Cell 60, 231-41 (2015); Brady, J.P. et al. Proc Natl Acad Sci U S A 114, E8194-E8203 (2017)). This increased disorder in de-mixed phases is also in agreement with a recent study that described the different compaction of protein chains in the dilute and dense phases (Farag, M. et al. Nat Commun 13, 7722 (2022)). We additionally observed $\beta$-strand conformations, which appear exclusive for expanded PHOX2B. This $\alpha$- to $\beta$-sheet interconversion by peptide plane flipping, postulated as a key step in amyloid formation, has never been observed in condensed milieu to date. Intriguingly, despite the established relevance of $\beta$-sheet structures in protein deposits (Hughes, M.P. et al. Science 359, 698-701 (2018);), formation of polyAla $\beta$-sheet aggregates have only been observed under extreme experimental conditions (Polling, S. et al. Nat Struct Mol Biol 22, 1008-15 (2015);). Indeed, whereas the abundance of $\beta$-strand conformations correlates with the affluence and persistence of expanded PHOX2B condensates, remains to be established.

Phase separation predictors suggest that the regions flanking PHOX2B polyAla tract (rich in GPG motifs) show high tendency to phase separate (stickers), while the hydrophobic polyAla tract would act as a spacer. Therefore, it follows that conformational changes in the hydrophobic polyAla spacer would promote contacts within the stickers in the dense phases. This is somehow in disagreement with the premise stating that polyAla expansions trigger protein aggregation through coiled coil stabilization (Pelassa, I. et al. Hum Mol Genet 23, 3402-20 (2014)), but is in accordance with the role of polyQ-driven assemblies supporting Whi3 phase separation (Zhang, H. et al. Mol Cell 60, 220-30 (2015)). While coiled coils are prevalent in proteins that phase separate, no evidence for coiled coil formation in the polyAla tract was inferred from our CD data. Interestingly, NMR relaxation data, obtained at higher protein concentrations than CD, showed that polyAla segments trigger oligomeric assembly regardless of the temperature (probably through coiled-coil formation). Although this property may ultimately lead to protein aggregation, it is not sufficient to explain the temperature-dependent phase transitions observed. Indeed, increasing the ionic strength have no effect on phase separation, but rather promote aberrant aggregation by hydrophobic collapse. Relaxation data also showed a stronger tendency of the PARMs to assemble into small oligomers, which could not be explained on the basis of the major $\alpha$-helical conformation of the extended polyAla tract. In addition, significant $\alpha$-helix to disordered conversions are observed coupled to phase transitions in expanded PHOX2B, in accordance with the decrease in thermal stability induced by PARMs (Pirone, L. et al. FEBS J 286, 2505-2521 (2019)). Disordered to $\alpha$-helix conversions are established as early steps in amyloid aggregation. However, the switch from $\alpha$-helix to disordered conformations observed for PARMs upon phase transitions could represent the polymorphism that is expected to occur in the condensate interface Therefore, the remarkable disorder in addition to the reduced condensate fluidity indicates that PHOX2B phase transitions are triggered by phase separation coupled to networking or percolation transitions. Considering that the abundant GPG stickers (Mittag, T. & Pappu, R.V. Mol Cell 82, 2201-2214 (2022)) in PHOX2B display significant polymorphism due to proline *cis-trans* isomerization, enriched disorder in the polyAla tract would increase networking during phase transitions. Therefore, our data indicate that the search of compounds that stabilize the major $\alpha$-helical structures and deter PARM-promoted nascent conformations would represent a reasonable rationale for the search of therapeutics for CCHS and other polyAla disorders. In particular, our data imply that the C-terminal region of polyAla $\alpha$-helix is significantly more dynamic, and could be the region to target the design of stabilizing compounds.

HSP70 and HSP90 chaperones primarily identify nascent conformations and impede PHOX2B phase transitions, providing a direct link between conformational transitions and phase de-mixing. We recently identified a chaperone tendency to recognize short structured motifs in disordered clients. Interestingly, here we show the selective chaperone arrest of minor disordered conformations in an otherwise structured segment of mutated PHOX2B. In particular, chaperones seize disordered conformations which could serve as secondary-structure templates priming fast phase transitions (Farag, M. et al. Nat Commun 13, 7722 (2022)). This selective identification is in agreement with the co-localization of HSP70 with the cytoplasmic aggregates formed by expanded PHOX2B, while it remained disperse in the cytosol in the presence of transfected wild-type PHOX2B (Trochet, D. et al. Hum Mol Genet 14, 3697-708 (2005)). In addition, up-regulation of HSP70-HSP40 machinery promotes the clearance of PARM aggregates (Bachetti, T. et al. Int J Biochem Cell Biol 39, 327-39 (2007)). Liquid-solid phase transitions are driven by the acquisition of β-sheet structure (Murray, D.T. et al. Cell 171, 615-627.e16 (2017); Hughes, M.P. et al. Science 359, 698-701 (2018)). Remarkably, the minor β metamorphic population observed in expanded PHOX2B is consistent with ThT negative, α-helical PHOX2B aggregates which are adhered by minimal β-sheet steric zippers (Pelassa, I. et al. Genet 23, 3402-20 (2014)). This peculiar sticking procedure would not be shared by other proteins undergoing phase separation and promoting ThT positive amyloid aggregates (Carrasco, J. et al. Nat Commun 14, 466 (2023)). For instance, chaperones promote TDP-43 LLPS and further amyloid aggregation to protect the cell against the prion-like behavior of soluble TDP-43, but inhibit PHOX2B phase transitions since it is the basis of its malfunction.

## 3. MATERIAL AND METHODS

### 3.1. Sample preparation

All the different human PHOX2B (UniProt KB Q99453, Origene) cDNA constructs were subcloned into modified pET28a vectors (Novagen) containing Thioredoxin (TXA) as a fusion protein followed by a six-histidine tag for $Ni^{2+}$ affinity purification and a Tobacco Etch Virus (TEV) protease cleavage site. Construct nomenclature refers to the length of the contained sequence (XL, L, S and XS; being XL the full-length protein) followed by the number of alanines in the polyAla tract (20, 23 and 26). All cloning procedures were performed following the Gibson Assembly method (New England Biolabs). PolyAla expanded mutants were generated by mutagenic PCR. XS20 and XS26 were subsequently used as templates for the addition of a C-terminal cysteine residue (C315) for the covalent attachment of a fluorescent tag (detailed below). Human HSP72 and HSP90β (named HSP70 and HSP90, respectively, for simplicity; Uniprot ID P54652 and P08238, respectively) cDNA sequences were cloned into respective pET28a vectors (GenScript). All sequences were verified by DNA sequencing. Cloning procedures were performed with the oligonucleotides (primers) included in Table 2 in both DH5α and XL1 *E. coli* strains:

Table 2. Primers for cloning in both DH5α and XL1 *E. coli* strains.

|  |  | Forward (5'-> 3') | Reverse (5'-> 3') |
|---|---|---|---|
| **XL20 insert** |  | ATTTCCAGGGATCCATGTAT AAAA TGGAATATTC (SEQ ID NO: 1) | GTGGTGCTCGAGTTAGAACATAC TGCTC (SEQ ID NO: 2) |
| **XL20 vector** |  | CTCGAGCACCACCACCACCA CTG  (SEQ ID NO: 3) | GACATGGATCCCTGGAAATACAG GTTTTC (SEQ ID NO: 4) |
| **L20 insert** |  | ATTTCCAGGGATCCATCCGC ACCACCTTCAC (SEQ ID NO: 5) | GTGGTGCTCGAGTTAGAACATAC T GCTC (SEQ ID NO: 6) |
| **S20 insert** |  | ATTTCCAGGGATCCGCTAAG TTTCGCAAGC (SEQ ID NO: 7) | GTGGTGCTCGAGTTAGAACATAC T GCTC (SEQ ID NO: 8) |
| **S20 vector** |  | CTCGAGCACCACCACCACCA CTG (SEQ ID NO: 21) | CTTAGCGGATCCCTGGAAATACA G GTTTTC (SEQ ID NO: 9) |
| **XS20 insert** |  | ATTTCCAGGGATCCGGCCCG GGGGGCCCGGG (SEQ ID NO: 10) | GTGGTGCTCGAGTTAGAACATAC T GCTC (SEQ ID NO: 11) |

(continued)

| | Forward (5'-> 3') | Reverse (5'-> 3') |
|---|---|---|
| XS20 vector | CTCGAGCACCACCACCACCA CTG (SEQ ID NO: 12) | GGGCCGGATCCCTGGAAATACAG GTTTTC (SEQ ID NO: 13) |
| XS23 | GCAAGGGCGGTGCAGCAGC AGCG GCTGCGGCTG (SEQ ID NO: 14) | CAGCCGCAGCCGCTGCTGCTGCA CCGCCCTTGC (SEQ ID NO: 15) |
| XS26 | GCAAGGGCGGTGCTGCAGC AGCA GCAGCAGCG (SEQ ID NO: 22) | CGCTGCTGCTGCTGCTGCAGCAC CGCCCTTGC (SEQ ID NO: 16) |
| C315 | GAGCAGTATGTTCTGTTAAC TCGAGCACCACC (SEQ ID NO: 23) | GGTGGTGCTCGAGTTAACAGAAC ATACTGCTC (SEQ ID NO: 17) |

PHOX2B fragments (including WT and PARMs) were produced in BL21 star (DE3) *E. coli* strain. Cells were cultured in rich media until $OD_{600nm} = 0.6$. For $^{15}N/^{13}C$ isotopic labeling, cultures were then harvested by a 20-min centrifugation at 5,300 g and the pellets resuspended in ¼ of the initial volume in M9 medium supplemented with 4g/l of $^{13}C$-glucose and 1 g/l of $^{15}NH_4Cl$ as the sole sources of C and N, respectively. Cultures were incubated at 37°C during 1-1,5h for the adaption to the new media until the optical density increased one more unit than the initial value after resuspension. Protein expression (both in rich and minimal media) was induced by the addition of 0.5 mM (0.1 mM for L20 production) isopropyl β-d-1-thiogalactopyranoside (IPTG) for 16-20 h at 25 °C. Cells were harvested by centrifugation at 5,300 g during 25 minutes at 4°C and resuspended in 30 ml/L of lysis buffer (50 mM KPi/500 mM NaCl/10 mM imidazole/2mM β-mercaptoethanol [pH 8]), including 2 μl of protease inhibitors (ThermoFisher Scientific), 0.08 mg/ml of DNAse I (Sigma-Aldrich) and 1 mg/ml lysozyme (Sigma-Aldrich) and sonicated. Subsequently, a clarification step at 4,000 g for 20 minutes at 4°C in an Optima XPN-90 Ultracentrifuge (Beckman Coulter) was performed to remove the insoluble debris.

The clarified lysates were loaded onto pre-equilibrated $Ni^{2+}$ affinity columns (Cytiva), adding 500 mM imidazole to the buffer for the elution (under the application of gradient elutions). TEV digestion was performed in 5 mM KPi/10 mM NaCl/10 mM imidazole/2mM β-mercaptoethanol [pH 8], by an overnight incubation at 4°C with 0.51 mg/ml of TEV protein (produced in house) per 15 mg of fusion protein. Cleaved PHOX2B protein constructs were directly cleared with a $Ni^{2+}$ affinity purification as previously detailed, where PHOX2B was present in the flowthrough fractions. Samples were further purified by a serial anion and cation exchange chromatography using Hitrap Q HP and SP HP columns (Cytiva), respectively, using 5mM KPi/10mM NaCl/2mM β-mercaptoethanol [pH 6.8]. Up to 1 M NaCl was added in gradient to the buffer for sample elution. Highly pure samples were concentrated below 60 μM using Vivaspin® Turbo 15 (3 MWCO) ultrafiltration tubes (Sartorius) in 5mM KPi/10mM NaCl/2mM β-mercaptoethanol/0,03 % sodium azide [pH 6.8] (NMR buffer) and snap-frozen at -80°C.

HSP70 and HSP90 molecular chaperones were produced in BL21 (DE3) and Rosseta 2 (DE3) star *E. coli* strains, respectively, using 1 mM IPTG for induction at $OD_{600} = 0.8-0.9$ during 4 h at 37°C. Cells were harvested and lysed in 20 mM Tris-HCl/500 mM NaCl/10 mM imidazole [pH 8] as explained previously. Recombinant proteins in the soluble fraction were purified by $Ni^{2+}$ affinity chromatography using high density $NiSO_4$ agarose beads (ABT), 20 mM Tris-HCl/500 mM NaCl/10 mM imidazole [pH 8] as binding buffer and binding buffer including 500 mM imidazole for elution. Proteins were further purified by size exclusion chromatography using HiLoad™ 26/60 Superdex™ 75 pg columns (Cytiva) in 10 mM Hepes/500 mM KCl/5 mM DTT [pH 7.5]. Fractions containing pure monomeric protein were pooled, concentrated using Vivaspin® 20 ultrafiltration tubes (10 MWCO, Sartorius) and stored at -80°C. Chaperone samples were buffer exchanged before the experiments into 20 mM Hepes/5 mM $MgCl_2$/10 mM KCl/1 mM DTT [pH 6.8] using Zeba spin desalting columns (ThermoFisher).

XS20 and XS26 C315 mutants were tagged with ATTO-488 and ATTO-565 (ATTOTEC GmbH) following the manufacturer protocol for fluorescent microscopy assays. After tagging, the excess of dye was removed with a size exclusion chromatography, using a Superdex ™ 75 10/300 GL column (Cytiva). An average 45% tagging efficiency was confirmed spectrophotometrically.

## 3.2. Turbidimetry assays

Protein samples of pure XS20, XS23, XS26 and L20 were prepared at 0.225 mM concentration in 5 mM KPi/10 mM NaCl/1 mM DTT/1mM benzamidine/0,03% sodium azide [pH 6.8] (incubation buffer). To monitor turbidity in presence of HSP70 and HSP90, 0.225 mM of PHOX2B proteins were incubated with 0.1 mM of the chaperones in 20 mM Hepes/10mM NaCl/5mM $MgCl_2$/2mM ATP/1 mM DTT/0,03% sodium azide [pH 6.8]. Turbidity was measured at 25°C using $Abs_{600}$ in a FLUOstar Omega (BMG LABTECH) multiwell plate reader using 96-well flat-bottom plates (Porvair Sciences). Measurements at 37°C, including 300 mM NaCl and 10% (w/v) PEG 4000 (Fisher Scientific) were performed using the same buffer and protein concentration as mentioned above. Samples were incubated for 84 hours with 60 s of agitation at 100 rpms before each measurement point (10 minutes between measurement points). All data was technically and biologically replicated, and presented as the average between replicates with the standard deviation.

## 3.3. Fluorescent microscopy

Fluorescent microscopy images were obtained in a Nikon Eclipse TE2000-U inverted microscope, a Nikon Ti2e-AXR-DUX-ST superresolution confocal microscopy, a Leica AF6000 LX and a Leica TCS SP8 STED confocal microscope. Protein samples were incubated in NMR buffer ranging from 0.1-0.25 mM protein concentration. Samples were first incubated at 37°C for 30 seconds, placed for 3 minutes at 4°C and finally incubated again at 37°C for the corresponding period of time. Fluorescent labeled XS20 and XS26 (both labeled with ATTO-488 and ATTO-565) were mixed with unlabeled samples at a molar ratio 1:400-1:600 before exchange into NMR buffer. Colocalization experiments were performed using 0.1 mM (1:400) XS26-ATTO488 and 0.15 mM (1:600) XS20-ATTO-565 incubated at 37°C for 3,5 minutes. To study PHOX2B condensates in presence of chaperones, 0.1 mM PHOX2B proteins were incubated with 0.1 mM chaperones (1:1 ratio) in 20 mM Hepes/10mM NaCl/5mM $MgCl_2$/2mM ATP/1 mM DTT/0,03 % sodium azide [pH 6.8] buffer. Samples were spotted onto glass slides (ThermoFisher scientific) and imaged upright.

Confocal FRAP experiments were performed on a home-built confocal microscope based on an Olympus IX-71 inverted microscope. The ATTO-488 labeled samples were excited by a 532 nm pulsed laser (LDH-P-FA-530B, PicoQuant GmbH) at a repetition rate of 40 MHz. The laser is coupled into a single mode fiber (P3-488PM-FC, Thorlabs GmbH) to obtain a Gaussian beam profile. A linear polarizer (LPVISE100-A, Thorlabs GmbH) and a quarter-wave plate (AQWP05M-600, Thorlabs GmbH) is used to obtain circular polarized light. The light is focused onto the sample using an oil immersion objective (UPLSAPO100XO, NA 1.40, Olympus Deutschland GmbH) and the position of the sample is adjusted using a piezo stage (P-517.3CD, Physik Instrumente (PI) GmbH & Co. KG) and controller (E-727.3CDA, Physik Instrumente GmbH & Co. KG). The emitted light is separated from the excitation beam by a dichroic beam splitter (zt532/640rpc, Chroma) and focused onto a 50 μm diameter pinhole (Thorlabs GmbH). After the pinhole, the emission signal is directed by a dichroic beam splitter (640 LPXR, Chroma) into a green (Brightline HC582/75, AHF; RazorEdge LP 532, Semrock) detection channel. Emission is focused onto avalanche photodiodes (SPCMAQRH- 14-TR, Excelitas) and the signals are registered by a time-correlated single photon counting (TCSPC) unit (HydraHarp400, PicoQuant). The setup is controlled by a commercial software package (SymPhoTime64, Picoquant GmbH). The surface was scan using a power of 1 μW (as measured at the entrance of the microscope body) to detect the condensates. To bleach part of the condensate, the confocal spot was focused nearby the condensate and the power increased to 175 μW for 2 seconds. For the preparation of the microscopy chamber, SecureSeal Hybridization chambers (2.6 mm depth, Grace Bio-Labs, USA) were glued on UV-Ozone cleaned (PSD-UV4, Novascan Technologies, USA) microscope coverslips. The surface was further cleaned with 1 M KOH for 15 min. After that, the chambers were washed three times with water and three times with PBS buffer. The surface was finally passivated with BSA-biotin for at least 1 hour. Right before the experiments, the BSA was removed and the chamber washed three times with NMR buffer. Protein samples were incubated as mentioned below.

TIRF-based FRAP measurements and time evolution observation of the condensates were performed on a commercial Nanoimager S microscope (ONI Ltd., UK) under TIRF illumination. Blue excitation was applied at 488 nm to excite the ATTO-488 labeled proteins. The auto focus of the setup was used to maintain the imaged samples on focus. Protein sample concentration, mixtures and incubation protocols were as mentioned above. Incubated samples were diluted 1/30 with NMR buffer in the microscopy chamber. Measurements were performed at 30°C. In order to standardize the measurements, data acquisition was started 6 min after the last 37°C incubation step. To study the time evolution of the condensates, observations (two frame video with 100 ms integration time per frame and 0.2 mW blue excitation) were performed at 6, 12, 18, 24 and 30 min. In each observation, 25 field of views were acquired in a rectangular pattern (5 x 5) covering 117 μm × 117μm. Duplicates were done for all the conditions tested, and averaged data is presented including standard deviation. To avoid being biased by diffraction limit, only condensates equal or larger than 2 pixels (each pixel being 117 nm) were included in the analysis. The following light program was set for FRAP measurements: i) Pre-bleaching observation (two frame video with 100 ms integration time per frame and 0.2 mW of blue excitation). ii) Bleaching (2.5 s of blue excitation at 169 mW power). iii) After-bleaching observation (two frame video with 100 ms integration time per frame and 0.2 mW blue excitation). iv) 360 s of recovery step without light excitation. v) Recovery after photobleaching observation (two frame video with 100 ms integration time per frame and 0.2 mW blue excitation).

TIRF videos were analyzed using ImajeJ. Only the first frame of the video was analyzed to avoid significant photobleaching of the field of view during the observation. The background of the images was subtracted using a rolling ball of 50 pixels radius. Condensates were analyzed using the Analyze Particles routine, which automatically identifies the condensates and calculates their intensity and size. For the quantification, the images were binarised (16-bit) and thresholded using the Otsu algorithm. Only properly focused condensates were included in the analyses, which contained information on particle area and fluorescence intensity.

### 3.4. NMR assignments, $^{15}$N Relaxation analysis and Structure Calculation

All the NMR spectra were acquired in an 800 MHz ($^1$H) Bruker AVNEO spectrometer equipped with a Z-gradient cryoprobe. $^1$H chemical shifts were referenced to the internal reference sodium trimethylsilylpropanesulfonate (DSS), and $^{15}$N and $^{13}$C chemical shifts were referenced indirectly to $^1$H using the corresponding gyromagnetic ratios. NMR assignments for XS20 were obtained at 5°C and 25°C using 0.5 mM of $^{15}$N/$^{13}$C-labeled XS20 in NMR buffer. The following NMR spectra were acquired to obtain unambiguous assignments at both temperatures: 2D $^{15}$N- and $^{13}$C-HSQCs, 2D CON, 3D HNCO, HN(CA)CO, HNCA, CBCA(CO)NH, hCC(CO)NH (15 ms mixing time) and HBHA(CO)NH. Sequential connectivities were obtained by high-resolution 3D HNNH spectra (using >90 points in F1 dimension), which enabled the unambiguous assignment of the polyAla segments even for the highly overlapped regions of the spectra. The C-terminal residues from the XS constructions (namely residues 307-314) were not assigned in the spectra. HNHA and $^{15}$N-NOESY-HSQC and $^{13}$C-NOESY-HSQC (using 100 and 120 ms mixing time, respectively) were used to obtain J-couplings and NOEs for structure calculation. Assignments for XS20 at both temperatures were deposited in the BMRB database (codes 51978 and 51979, respectively). Additional XS20 samples were incubated up to 14 days at 25°C with no agitation in incubation buffer before the NMR experiments. Only minor sample degradation within NMR incubation times was detected by mass spectrometry and gel electrophoresis. Assignment of 0.5 mM $^{15}$N/$^{13}$C-labeled XS23 and XS26 in NMR buffer were obtained at 25°C by means of 2D $^{15}$N- and $^{13}$C-HSQCs, 2D CON, 3D HNCO, HN(CA)CO, HNCA, CBCA(CO)NH and HNNH spectra. Additional samples of XS23 and XS26 were incubated for 14 and 7 days at 25°C in incubation buffer with no agitation, respectively. Sequential connectivities were lost for the incipient crosspeaks appearing in the $^{15}$N-HSQC XS23 and XS26 spectra upon incubation. However, 3D CBCA(CO)NH spectra revealed that the large majority of those peaks belonged to residues preceded by Ala residues in disordered conformation, due to their characteristic $C\alpha$ and $C\beta$ chemical shifts. Therefore, the new crosspeaks in the [15]NHSQC were assigned following arbitrary numbering, which was maintained in the nascent crosspeaks detected in $^{13}$C-HSQC and CACO spectra. In addition, alanine residues from the central section of the polyAla $\alpha$-helix (residues 253-258 in particular) showed strong signal overlap in the H-N correlations, which enabled to observe nascent crosspeaks in the 3D CBCA(CO)NH spectra with chemical shifts corresponding to alanines in alternative conformations. These disordered polyAla crosspeaks were also observed in 2D $^{13}$C-HSQC spectra, which displays the whole palette of conformation-dependent C-H correlations. The nascent alanine moieties were reproduced in different protein batches upon incubation. 0.5 mM $^{15}$N/$^{13}$C-labeled L20 was used to validate the structure of XS20 using 2D $^{15}$N- and $^{13}$C-HSQCs, 2D CON, 3D HNCO and CBCA(CO)NH spectra at 5°C.

Loss of NMR signal intensity was calculated comparing the $^{15}$N-HSQC NMR signal intensities for freshly prepared XS20 samples at 0.5 mM and 25°C *vs.* the NMR signal intensity for XS20 samples which were incubated at 25°C. For CACO spectra, signal intensity for 0.225 mM XS26 incubated in incubation buffer for 48 h was compared to that for fresh XS26. Secondary chemical shifts were calculated as $\Delta C\alpha$-$\Delta C\beta$, where $\Delta C\alpha$ and $\Delta C\beta$ are the differences between experimentally obtained $C\alpha$ and $C\beta$ chemical shifts at the specific protein concentrations and computed shifts for disordered PHOX2B constructs at the same temperature and pH (https://spin.niddk.nih.gov/bax/nmrserver/Poulsen rc CS/). $^3J_{HNHA}$ coupling constants were calculated on the basis of 3D HNHA experiments. $^{15}$N longitudinal ($R_1$), rotating frame ($R_{1\rho}$) and ($^1$H)-$^{15}$N NOE relaxation data for 0.5 mM XS20, XS23 and XS26 samples in NMR buffer were obtained using standard Bruker pulse sequences acquired at 25°C (or 5°C and 25°C for XS20) in the 800 MHz spectrometer. In all relaxation experiments the spectral width was 12500 Hz for $^1$H and 2027 Hz for $^{15}$N dimensions. Eight relaxation delays (20; 60; 100; 240; 600; 1000; 1600 and 2400 ms) were used for $^{15}$N $R_1$ measurements, as well as to measure $^{15}$N $R_{1\rho}$ values (using 8.00; 35.99; 75.99; 100.00; 156.00; 200.00; 347.99 and 699.99 ms). Relaxation values and uncertainties were calculated by fitting an exponential decay to the data. Het-NOEs were calculated from the ratio of cross peak intensities in spectra collected with and without amide proton saturation during the recycle delay. Uncertainties in peak heights were determined from the standard deviation of the intensity distribution in signal-less spectral regions. All NMR spectra were processed in Topspin 4.1.1 and analyzed in Sparky.

The $S^2$ order parameter was derived from the chemical shifts obtained by TALOSN. The overall correlation time ($\tau_c$) was estimated from the ratios of the mean values of $T_1$ and $T_2$ as $\tau_c \simeq = 1 / (4\,\pi * $ $^{15}$N frequency in Hz$) * ((6* R_2/R_1) - 7))^{1/2}$ which was derived from eqn. 8 of Kay *et al* (Kay, L.E., et al. Biochemistry 28, 8972-9 (1989)) that considers J(0) and J(w) spectral densities and discounts terms of higher frequencies from a subset of residues with little internal motion and no significant exchange broadening. This subset excluded residues with $T_2$ values lower than the average minus one standard deviation, unless their corresponding $T_1$ values were larger than the average plus one standard deviation. Experimental $\tau_c$ values for XS20 at 5°C and 25°C were compared to the correlation time calculated on the basis of its size and shape in solution, using HydroNMR. The atomic structures for the fragment 241-260 of PHOX2B at both temperatures (pure $\alpha$-helix) were considered in the calculations, and $\tau_c$ values were averaged for the ensemble structures. Temperature dependent solvent viscosity was considered in the calculations, applying 3.0 Å of atomic element radius. 3D coordinates of canonical $\alpha$-helices for 20, 23 and 26 alanines were also generated using the builder module in PyMOL (PyMOL Molecular Graphics System, Version 1.8.x Schrödinger, LLC.). HydroNMR was applied to the generated $\alpha$-helices, which reported the following $\tau_c$ averaged values (in ns): 1,85 (XS20 at 5°C), 1,05 (XS20 at 25°C), 1,24 (XS23 at 25°C) and 1,43 (XS26 at 25°C). These values indicate an increase of 0,19 ns in $\tau_c$ every 3 additional alanines, which is significantly smaller than the experimental values obtained for the extended variants. These discrepancies, in addition to the difference between experimental and calculated $\tau_c$ for XS20 structure, indicate that PHOX2B has a tendency to assemble in solution through the polyAla helix, and that PARMs increase these associations. Relaxation and correlation time values are shown in Table 3.

Table 3. Correlation time and relaxation parameters for PHOX2B constructs.

| | | | | $R_1$ (s-1) | | $R_2$ (s-1) | HETNOE | $\tau_C$ (ns) |
|---|---|---|---|---|---|---|---|---|
| | ALL | AAA | ALL | AAA | ALL | AAA | | |
| XS20 (5°C) | 1,89 +/-0,40 | 1,54 +/-0,19 | 6,1 +/-4,5 | 11,8 +/-2,7 | 0,42 +/-0,21 | 0,67 +/-0,08 | 6,7 (1,7) |
| XS20 (25°C) | 1,62 +/-0,31 | 1,83 +/-0,25 | 3,5 +/-2,0 | 5,9 +/-1,2 | 0,12 +/-0,40 | 0,53 +/-0,18 | 3,6 (0,9) |
| XS23 (25°C) | 1,60+/- 0,23 | 1,73+/- 0,13 | 3,7+/- 2,1 | 6,3+/- 1,1 | 0,11 +/-0,47 | 0, 59+/- 0,18 | 4,0 (NA) |
| XS26 (25°C) | 1,75+/- 0,24 | 1,88+/- 0,21 | 4,9+/- 3,4 | 8,9+/- 1,9 | 0,18+/- 0,50 | 0,65+/- 0,12 | 4,7 (NA) |
| XS23i (25°C) | 1,40+/- 0,42 | 1,11+/- 0,34 | 2,9+/- 1,5 | 2,1+/- 2,0 | -1,18+/- 0,90 | - 0.77+/- 0, 83 | - |

Data is presented considering either the whole sequence (ALL) or only the polyAla segment (AAA). Data is shown as calculated value +/- SD. Correlation time ($\tau_c$) is presented based on experimental R1 and R2 rates of the polyAla segments, and based on theoretical calculations using HydroNMR on monomeric, experimentally-derived XS20 $\alpha$-helical structures (presented between parenthesis). HydroNMR-based theoretical $\tau_c$ were not calculated for XS23 and XS26 due to the lack of atomic structure for these constructs. The difference in $\tau_c$ for XS20 at 5°C and 25°C is consistent with the increase of solvent viscosity at lower temperatures. However, the disparity between experimental and theoretical $\tau_c$ values indicates that PHOX2B is assembling into oligomeric species, regardless of the temperature. HydroNMR postulates an increase in $\tau_c$ of 0,19 ns for a canonical $\alpha$-helix upon addition of 3 residues. Therefore, the significant difference in $\tau_c$ for the expanded mutants, more especially for XS26, indicates the higher propensity to associate for the PARMs. XS23i refers to the relaxation data for incubated XS23 (Fig. 14). For XS23i, AAA refers to the nascent Ala moieties. Although no $\tau_c$ was calculated for XS23i due to the lack of structured elements, relaxation data clearly indicates that the emerging Ala belong to highly flexible fragments.

The NMR structure of XS20 at 5°C and 25°C was calculated with the program CYANA v3.98.13 based on experimental NOE-derived distance constraints and TalosN-derived dihedral constraints following the standard 7-cycle iterative process and a final annealing using the list of restraints obtained in the last cycle. One hundred structures were generated using the mentioned procedure. Structural statistics for the calculated structures are summarized in Table 4. $\alpha$-helical boundaries were experimentally validated by the observation of i+3 and i+4 H-H NOEs. The 20 conformers with the lowest target function values were selected and deposited in the Protein Data Bank under the accession numbers 8PTL and 8PUI. The structural ensembles were visualized and examined using MolMol and Pymol v2.0 (PyMOL Molecular Graphics System, Version 2.0 Schrödinger, LLC.). PROCHECK-NMR version 3.4.4 was used to analyze the quality of the refined structures.

Table 4. Structure Calculation Statistics for the 3D NMR XS20 structures at 5°C and 25°C.

| XS20 @ 5°C | |
|---|---|
| NOE distance constraints: | |
| Intraresidual | 52 |
| Medium range (within 5 residues) | 5 |
| Long range (5 or more residues) | 12 |
| Dihedral angle restraints | 68 |

(continued)

| XS20 @ 5°C | |
|---|---|
| Stereospecific 1H assignments<br>Total | 1<br>138(6.9)* |
| CYANA target energy function(Å2) | $0.06 \pm 0.04$ |
| RMSD (to mean coordinates) 241-260:<br>backbone(Å)<br>All heavy atoms(Å) | <br>1.41<br>1.63 |
| Procheck Ramachandran plot statistics:<br>Most favored regions (%)<br>Additionally allowed favored regions (%) | <br>77.5<br>22.5 |
| | |
| XS20 @ 25°C | |
| NOE distance constraints: | |
| Intraresidual | 38 |
| Medium range (within 5 residues) | 6 |
| Long range (5 or more residues) | 0 |
| Dihedral angle restraints | 48 |
| Stereospecific 1H assignments | 0 |
| Total | 92(4.6)* |
| CYANA target energy function(Å2) | $0.17 \pm 0.03$ |
| RMSD (to mean coordinates) 241-260: | |
| backbone(Å) | 1.08 |
| All heavy atoms(Å) | 1.29 |
| Procheck Ramachandran plot statistics: | |
| Most favored regions (%) | 76.3 |
| Additionally allowed favored regions (%) | 23.7 |
| *only for the 20 Alanine residues | |

NMR titrations were based on 2D $^{15}$N-HSQC, $^{13}$C-HSQC and CACO spectra measured at 25°C in the 800 MHz spectrometer in 20 mM Hepes/10mM NaCl/5mM MgCl$_2$/2mM ATP/1 mM DTT/0,03 % sodium azide [pH 6.8] buffer. 0.07 mM (for the $^{15}$N-HSQC spectra), 0.1 mM (for the $^{13}$C-HSQC spectra) and 0.225 mM (for the CACO spectra). $^{15}$N/$^{13}$C-labeled XS26 were mixed with fresh 0.14 mM unlabeled (therefore, NMR invisible) HSP70 and HSP90 (for the $^{15}$N-HSQC-based titrations) or 0.1 mM fresh HSP70 (for the $^{13}$C-HSQC-based titrations), while CACO samples were incubated 48 h with no agitation at 25°C with 0.45 mM HSP70. The data shown are the signal intensity decay for the interactions, which is obtained comparing the normalized NMR signal intensity for the complex ratios and the intensity for XS26 alone at the same concentration. For the $^{13}$C-HSQC-based titrations, values shown are averaged between the C$\alpha$ and C$\beta$ crosspeak intensities, and include alternative conformations both for polyAla and nascent alanine moieties.

## 3.5. Circular Dichroism (CD)

CD spectra were recorded at 25°C before and after thermal denaturation at 95°C in a J-810 Jasco polarimeter using a 0.1 cm cuvette and 0.265 mg/ml (for XS20 and XS26) and 0.132 mg/ml (for XS23) protein concentration. The sample buffer was 5 mM KPi/10 mM NaCl /1mM DTT/0,01% azide [pH 6.8]. Samples were serially diluted in water to minimize signal intensity. Thermal unfolding was induced by heating the samples at 95°C for 15 minutes followed by a 6 minutes cool down to 25°C. For each measurement, ten scans were averaged and, after correction for the buffer contribution, transformed into mean residue weight ellipticity ($\Theta \cdot$ 10-3 (degree $\cdot$ cm2 $\cdot$ dmol-1)) as follows:

$$Molar\ Ellipticity = \frac{\theta/10\ x\ Optical\ path\ lenght\ (cm <)\ x\ Concentration\ (M)}{residue\ number - 1}$$

**Claims**

1. An *in vitro* method for the screening of active agents that inhibit changes in secondary structure in a transcription factor, wherein the changes in secondary structure are responsible for the formation of aggregates of said transcription factor, wherein said method comprises the following steps:

   a) determining by NMR spectroscopy any change in secondary structure in a transcription factor, or a fragment thereof,

   - wherein said transcription factor comprises an amino acid repeat mutation,
   - wherein the amino acid repeat mutation gives rise to changes at the secondary structure level, and
   - wherein the changes in secondary structure are responsible for the formation of aggregates,

   b) determining by NMR spectroscopy any change in secondary structure in a transcription factor used in step a), or a fragment thereof, upon incubation with an active agent, and

   wherein the active agent is selected as an inhibitor of the changes in secondary structure that are responsible for the formation of aggregates, if there is a reduction in the changes in secondary structure determined in step b) compared to the changes in secondary structure determined in step a).

2. Method according to claim 1, further comprising a step c) of determining the formation of aggregates of the transcription factor, or a fragment thereof, of the step a) and step b).

3. Method according to claim 2, wherein step c) is carried out by light scattering, optical microscopy, electron microscopy, atomic force microscopy, analytical ultracentrifugation, chromatography and/or mass spectrometry.

4. Method according to any one of the claims 1 to 3, wherein the transcription factor comprises an amino acid repeat mutation of a hydrophobic aliphatic amino acid or a hydrophilic polar uncharged amino acid.

5. Method according to claim 4, wherein hydrophobic aliphatic amino acid is alanine, or wherein the hydrophilic polar uncharged amino acid is glutamine.

6. Method according to any one of claims 1 to 5, wherein the amino acid repeat comprises at least 10 consecutive identical residues; preferably wherein the amino acid expansion comprises from 10 to 250 consecutive identical residues.

7. Method according to any one of claims 1 to 6, wherein the transcription factor is a developmental transcription factor.

8. Method according to any one of claims 1 to 7, wherein the transcription factor is a developmental transcription factor selected from the list consisting of ATN1, HTT, JPH3-AS, AR, ATXN1, ATXN2, ATXN3, CACNA1A, ATXN7, ATXN8, TBP, PABPN1, ARX, FOXL2, RUNX2, PHOX2B, HOXA13, ZIC2, HOXD13, ARX and SOX3.

9. Use of a transcription factor that comprises an amino acid repeat mutation, or a fragment thereof, for the *in vitro* screening of an active agent that inhibits changes in secondary structure in the transcription factor by NMR spectroscopy, wherein the changes in secondary structure are responsible for the formation of aggregates of said transcription factor.

10. Use according to claim 9, wherein the transcription factor comprises an amino acid repeat mutation of a hydrophobic aliphatic amino acid or a hydrophilic polar uncharged amino acid.

11. Use according to claim 9 or 10, wherein the hydrophobic aliphatic amino acid is alanine or wherein the hydrophilic polar uncharged amino acid is glutamine.

**12.** Use according to any one of claims 9 to 11, wherein the amino acid repeat comprises at least 10 residues; preferably wherein the amino acid repeat comprises from 10 to 250 residues.

**13.** Use according to any one of claims 9 to 12, wherein the transcription factor is a developmental transcription factor.

**14.** Use according to any one of claims 9 to 13, wherein transcription factor is a developmental transcriptional factor selected form the list consisting of ATN1, HTT, JPH3-AS, AR, ATXN1, ATXN2, ATXN3, CACNA1A, ATXN7, ATXN8, TBP, PABPN1, ARX, FOXL2, RUNX2, PHOX2B, HOXA13, ZIC2, HOXD13, ARX and SOX3.

**A**

PHOX2B

HOMEOBOX    PARMS

XL

1    15  30      101    151  167      241 260        314

L

101                                          314

S

151                            314

XS

228                  314

**B**

230              240              250              260
GPG GPGGEPGKGG AAAAAAAAAA AAAAAAAAAA

270              280              290
GGLAAAGGPG QGWAPGPGPI TSIPDSLGGP

300              310
FASVLSSLQR PNGAKAALVK SSMF

XS20

(SEQ ID NO: 18)

Fig. 1

Fig. 1 (continu.)

Fig. 1 (continu.)

**Fig. 2**

Fig. 2 (continu.)

**Fig. 2 (continu.)**

Fig. 3

EP 4 556 905 A1

Fig. 4

Fig. 5

Fig. 6

**Fig. 7.**

Fig. 7 (continu.)

day 0 @ 25°C
day 14

Fig. 8.

**Fig. 9.**

Fig. 10.

Fig. 11.

Fig. 12.

Fig. 13.

Fig. 13 (contin.).

Fig. 14.

EP 4 556 905 A1

45

Fig. 15.

Fig. 16.

Fig. 17.

Fig. 18.

Fig. 19.

Fig. 20.

Figure 20 (continu.)

Fig. 21.

Fig. 22

Europäisches
Patentamt

European
Patent Office

Office européen
des brevets

# EUROPEAN SEARCH REPORT

**Application Number**

**EP 23 38 3165**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 02/41002 A2 (ALPHABETA AB [SE]; WHITE MARTIN PAUL [GB]; JOHANSSON JAN [SE]) 23 May 2002 (2002-05-23) * pg 2, l 15-pg 3, l 30; pg 8, l 9- pg 13, l 30; cl 1-8 * | 1-14 | INV. G01N33/00 G01N33/68 |
| Y | DAVID PANTOJA-UCEDA: "Conformational Priming of RepA-WH1 for Functional Amyloid Conversion Detected by NMR Spectroscopy", STRUCTURE, [Online] vol. 28, no. 3, 1 March 2020 (2020-03-01), pages 336-347.e4, XP093155025, AMSTERDAM, NL ISSN: 0969-2126, DOI: 10.1016/j.str.2019.12.007 Internet Retrieved from the Internet: URL:https://www.cell.com/structure/pdf/S09 69-2126(19)30446-0.pdf> [retrieved on 2024-04-29] * graphical abstract; summary; pg 337-right col, second par * | 1-14 | |

-/--

**TECHNICAL FIELDS SEARCHED (IPC)**

G01N

The present search report has been drawn up for all claims

| Place of search | Date of completion of the search | Examiner |
|---|---|---|
| Munich | 3 May 2024 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 23 38 3165

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TIZIANA BACHETTI: "Distinct pathogenetic mechanisms for PHOX2B associated polyalanine expansions and frameshift mutations in congenital central hypoventilation syndrome", HUMAN MOLECULAR GENETICS, [Online] vol. 14, no. 13, 1 July 2005 (2005-07-01), pages 1815-1824, XP093157083, GB ISSN: 0964-6906, DOI: 10.1093/hmg/ddi188 Retrieved from the Internet: URL:https://academic.oup.com/hmg/article-pdf/14/13/1815/1717700/ddi188.pdf> [retrieved on 2024-04-29] * abstract; pg 1817, left col, second par-pg 1819, right col, third par * | 8,14 | |
| Y | JUN-YE HONG: "Author Correction: Structural and dynamic studies reveal that the Ala-rich region of ataxin-7 initiates [alpha]-helix formation of the polyQ tract but suppresses its aggregation", SCIENTIFIC REPORTS, [Online] vol. 10, no. 1, 9 December 2020 (2020-12-09), XP093157127, US ISSN: 2045-2322, DOI: 10.1038/s41598-020-66680-9 Retrieved from the Internet: URL:https://www.nature.com/articles/s41598-020-66680-9> [retrieved on 2024-04-29] * abstract; pg 1-pg 2, third par, fig 1 * | 8,14 | |
| A | WO 2010/054127 A1 (ELAN PHARM INC [US]; CAMBRIDGE ENTPR LTD [GB] ET AL.) 14 May 2010 (2010-05-14) * pg 4 lines 15-29, pg 6; cl. 1-2, 25, 30, 33 * | 1-14 | TECHNICAL FIELDS SEARCHED (IPC) |

The present search report has been drawn up for all claims

| Place of search | Date of completion of the search | Examiner |
|---|---|---|
| Munich | 3 May 2024 | Motrescu-Hateley, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 38 3165**

This annex lists the patent family members relating to the patent documents cited in the above-mentioned European search report.
The members are as contained in the European Patent Office EDP file on
The European Patent Office is in no way liable for these particulars which are merely given for the purpose of information.

**03-05-2024**

| Patent document cited in search report | | Publication date | Patent family member(s) | | Publication date |
|---|---|---|---|---|---|
| WO 0241002 | A2 | 23-05-2002 | AT | E518140 T1 | 15-08-2011 |
| | | | AU | 2384302 A | 27-05-2002 |
| | | | CA | 2429180 A1 | 23-05-2002 |
| | | | EP | 1337855 A2 | 27-08-2003 |
| | | | US | 2002143105 A1 | 03-10-2002 |
| | | | US | 2005059084 A1 | 17-03-2005 |
| | | | US | 2010130431 A1 | 27-05-2010 |
| | | | US | 2014187747 A1 | 03-07-2014 |
| | | | WO | 0241002 A2 | 23-05-2002 |
| WO 2010054127 | A1 | 14-05-2010 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MATERA, I. et al.** *J Med Genet*, 2004, vol. 41, 373-80
- **CHAVALI, S.** ; **SINGH, A.K.** ; **SANTHANAM, B.** ; **BABU, M.M.** *Nature Reviews Chemistry*, 2020, vol. 4, 420-434
- **PIRONE, L. et al.** *FEBS J*, 2019, vol. 286, 2505-2521
- **ANJA MITTAG** ; **ROHIT V. PAPPU**. A conceptual framework for understanding phase separation and addressing open questions and challenges.. *Molecular Cell*, 2022, vol. 82 (12), 2201-221
- **ALDERSON TR** ; **KAY LE.** NMR spectroscopy captures the essential role of dynamics in regulating biomolecular function.. *Cell*, 04 February 2021, vol. 184 (3), 577-595
- **DI LASCIO, S. et al.** *Hum Mutat*, 2018, vol. 39, 219-236
- **PELASSA, I. et al.** *Hum Mol Genet*, 2014, vol. 23, 3402-20
- **CIANI, B.** ; **HUTCHINSON, E.G.** ; **SESSIONS, R.B.** ; **WOOLFSON, D.N.** *J Biol Chem*, 2002, vol. 277, 10150-5
- **BACHETTI, T. et al.** *Hum Mol Genet*, 2005, vol. 14, 1815-24
- **DI ZANNI, E. et al.** aggregates containing polyAla expanded PHOX2B proteins.. *Neurobiol Dis*, 2012, vol. 45, 508-18
- **DI LASCIO, S. et al.** *Neurobiol Dis*, 2013, vol. 50, 187-200
- **BASU, S. et al.** *Cell*, 2020, vol. 181, 1062-1079
- **TROCHET, D. et al.** *Hum Mol Genet*, 2005, vol. 14, 3697-708
- **PELASSA, I. et al.** *Genet*, 2014, vol. 23, 3402-20
- **LILLIU, E. et al.** *J Struct Biol*, 2018, vol. 204, 572-584
- **AMIEL, J. et al.** *Nat Genet*, 2003, vol. 33, 459-61
- **DUBREUIL, V. et al.** *Proc Natl Acad Sci U S A*, 2008, vol. 105, 1067-72
- **MENSAH, M.A. et al.** *Nature*, 2023, vol. 614, 564-571
- **BASU, S. et al.** Unblending of Transcriptional Condensates in Human Repeat Expansion Disease.. *Cell*, 2020, vol. 181, 1062-1079
- **LESTER, E. et al.** *Proc Natl Acad Sci U S A*, 2023, vol. 120, e2217759120
- **BAIAS, M. et al.** *J Am Chem Soc*, 2017, vol. 139, 1168-1176
- **ESCOBEDO, A. et al.** *Nat Commun*, 2019, vol. 10, 2034
- **BURKE, K.A.** ; **JANKE, A.M.** ; **RHINE, C.L.** ; **FAWZI, N.L.** *Mol Cell*, 2015, vol. 60, 231-41
- **BRADY, J.P et al.** *Proc Natl Acad Sci U S A*, 2017, vol. 114, E8194-E8203
- **FARAG, M. et al.** *Nat Commun*, 2022, vol. 13, 7722
- **HUGHES, M.P. et al.** *Science*, 2018, vol. 359, 698-701
- **POLLING, S. et al.** *Nat Struct Mol Biol*, 2015, vol. 22, 1008-15
- **ZHANG, H. et al.** *Mol Cell*, 2015, vol. 60, 220-30
- **MITTAG, T.** ; **PAPPU, R.V.** *Mol Cell*, 2022, vol. 82, 2201-2214
- **BACHETTI, T. et al.** *Int J Biochem Cell Biol*, 2007, vol. 39, 327-39
- **MURRAY, D.T. et al.** *Cell*, 2017, vol. 171, 615-627
- **CARRASCO, J. et al.** *Nat Commun*, 2023, vol. 14, 466
- **KAY, L.E. et al.** *Biochemistry*, 1989, vol. 28, 8972-9